# EUROPEAN PATENT SPECIFICATION

(11) **EP 4 144 405 B1**
(45) Date of publication and mention of the grant of the patent: **15.05.2024**
(21) Application number: 22193623.0
(22) Date of filing: 02.09.2022
(51) Int. Cl.: A61N 1/36

(54) **PHYSIOLOGICAL SIGNAL SENSING FOR CLOSED-LOOP STIMULATION**
PHYSIOLOGISCHE SIGNALERFASSUNG FÜR EINE STIMULATION MIT GESCHLOSSENEM REGELKREIS
DÉTECTION DE SIGNAUX PHYSIOLOGIQUES POUR STIMULATION EN BOUCLE FERMÉE

(30) Priority: 07.09.2021 US 202163241510 P; 08.08.2022 US 202217818246
(43) Date of publication of application: 08.03.2023
(73) Proprietor: Medtronic, Inc., Minneapolis, Minnesota 55432 (US)
(72) Inventor: Isaacson, Benjamin P., Minneapolis, 55432-3568 (US); Stanslaski, Scott R., Minneapolis, 55432-3568 (US); Becker, Abbey Beuning Holt, Minneapolis, 55432-3568 (US); Molina, Rene A., Minneapolis, 55432-3568 (US); Zarns, Caleb C., Minneapolis, 55432-3568 (US); Linde, David E., Bremerton, 98312 (US); Buse, Nicholas D., Minneapolis, 55432-3568 (US); Brink, Thaddeus S., Minneapolis, 55432-3568 (US)
(74) Representative: Zimmermann & Partner Patentanwälte mbB

(56) References cited:
- WO-A2-2014/189944
- US-A1- 2018 110 991
- US-A1- 2021 121 697
- FRANZ HELL ET AL: "Deep Brain Stimulation Programming 2.0: Future Perspectives for Target Identification and Adaptive Closed Loop Stimulation", FRONTIERS IN NEUROLOGY, vol. 10, 3 April 2019 (2019-04-03), XP055767160, DOI: 10.3389/fneur.2019.00314
- QIAN XING ET AL: "A Method for Removal of Deep Brain Stimulation Artifact From Local Field Potentials", IEEE TRANSACTIONS ON NEURAL SYSTEMS AND REHABILITATION ENGINEERING, vol. 25, no. 12, 1 December 2017 (2017-12-01), pages 2217-2226, XP093010180, USA ISSN: 1534-4320, DOI: 10.1109/TNSRE.2016.2613412

## Description

### TECHNICAL FIELD

This disclosure generally relates to a system and a computer readable storage medium for electrical stimulation therapy.

### BACKGROUND

Medical devices may be external or implanted, and may be used to deliver electrical stimulation therapy to various tissue sites of a patient to treat a variety of symptoms or conditions such as chronic pain, tremor, Parkinson's disease, other movement disorders, epilepsy, urinary or fecal incontinence, sexual dysfunction, obesity, or gastroparesis. A medical device delivers electrical stimulation therapy via one or more leads that include electrodes located proximate to target locations associated with the brain, the spinal cord, pelvic nerves, peripheral nerves, or the gastrointestinal tract of a patient. For bipolar stimulation, the electrodes used for stimulation may be on one or more leads. For unipolar stimulation, the electrodes may be on one or more leads, and an electrode on a stimulator housing located remotely from the target site (e.g., near the clavicle). It may be possible to use a leadless stimulator including electrodes mounted on the stimulation housing. Electrical stimulation is used in different therapeutic applications, such as deep brain stimulation (DBS), spinal cord stimulation (SCS), pelvic stimulation, gastric stimulation, or peripheral nerve field stimulation (PNFS).

A clinician may select values for a number of programmable parameters in order to define the electrical stimulation therapy to be delivered by the implantable stimulator to a patient. For example, the clinician may select one or more electrodes for delivery of the stimulation, a polarity of each selected electrode, a voltage or current pulse amplitude, a pulse width, and a pulse frequency as stimulation parameters. A set of parameters, such as a set including electrode combination, electrode polarity, amplitude, pulse width, and pulse rate, may be referred to as a program in the sense that they define the electrical stimulation therapy to be delivered to the patient. US 2021/121697 A1 and US 2018/110991 A1 disclose deep brain stimulation systems with closed-loop stimulation modes.

### SUMMARY

The invention is defined by independent claims 1 and 9. In the following all methods are shown for illustrative purposes only and do not form part of the invention. This disclosure describes example techniques for optimizing closed-loop therapy. In some examples of closed-loop therapy, a medical device senses biomarker signals (e.g., physiological signals generated from the patient's body), and determines therapy parameters based on the sensed biomarker signals. However, in some cases, the delivery of therapy may impact the ability to accurately sense the biomarker signals. For instance, the delivery of therapy may squelch the biomarker signals or interfere with the biomarker signals.

In one or more examples, a medical device may deliver therapy with the patient in two different patient states, and determine whether there is a difference in the biomarker signals when the patient is in the two different patient states. The medical device may be configured to select a therapy mode based on whether there is a difference in the biomarker signals when the patient is in the two different patient states. For instance, if there is no change in the biomarker signals, then there is a possibility that the therapy is impacting the ability to accurately sense the biomarker signal. In such cases, the medical device may deliver therapy in accordance with a polling closed-loop therapy mode, described in more detail below. If there is change in the biomarker signals, then there is a possibility that the therapy is not impacting the ability to accurately sense the biomarker signal. In such cases, the medical device may deliver therapy in accordance with a normal closed-loop therapy mode, described in more detail below.

The example medical devices described in this disclosure may provide improvement to the technology of therapy delivery. For instance, the example medical devices provide a practical application for therapy delivery that improves the overall operation of the medical devices.

In one example, this disclosure describes a system for electrical stimulation delivery that includes stimulation generation circuitry configured to deliver electrical stimulation to a patient; sensing circuitry configured to sense one or more biomarker signals; and processing circuitry configured to: cause the stimulation generation circuitry to deliver electrical stimulation with the patient in a first patient state; receive a first instance of a biomarker signal of the sensed one or more biomarker signals in presence of the electrical stimulation from the stimulation generation circuitry delivering the electrical stimulation with the patient in the first patient state; cause the stimulation generation circuitry to deliver electrical stimulation with the patient in a second patient state; receive a second instance of the biomarker signal of the sensed one or more biomarker signals in presence of the electrical stimulation from the stimulation generation circuitry delivering the electrical stimulation with the patient in the second patient state; determine whether a difference between the first instance of the biomarker signal and the second instance of the biomarker signal satisfies a threshold; select a therapy mode for electrical stimulation delivery based on whether the difference between the first instance of the biomarker signal and the second instance of the biomarker signal satisfies the threshold; and cause the stimulation generation circuitry to deliver electrical stimulation in accordance with the selected therapy mode.

In another example, which does not form part of the invention, this disclosure describes a method for electrical stimulation delivery that includes causing, with processing circuitry, a stimulation generation circuitry to deliver electrical stimulation with a patient in a first patient state; receiving, with the processing circuitry, a first instance of a biomarker signal sensed by a sensing circuitry in presence of the electrical stimulation from the stimulation generation circuitry delivering the electrical stimulation with the patient in the first patient state; causing, with the processing circuitry, the stimulation generation circuitry to deliver electrical stimulation with the patient in a second patient state; receiving, with the processing circuitry, a second instance of the biomarker signal sensed by the sensing circuitry in presence of the electrical stimulation from the stimulation generation circuitry delivering the electrical stimulation with the patient in the second patient state; determining, with the processing circuitry, whether a difference between the first instance of the biomarker signal and the second instance of the biomarker signal satisfies a threshold; selecting, with the processing circuitry, a therapy mode for electrical stimulation delivery based on whether the difference between the first instance of the biomarker signal and the second instance of the biomarker signal satisfies the threshold; and causing, with the processing circuitry, the stimulation generation circuitry to deliver electrical stimulation in accordance with the selected therapy mode.

In another example, this disclosure describes a system for electrical stimulation delivery that includes stimulation generation circuitry configured to deliver electrical stimulation to a patient; sensing circuitry configured to sense one or more biomarker signals; and processing circuitry configured to: cause the stimulation generation circuitry to deliver electrical stimulation having a first parameter set based on the patient being in a first patient state; determine that the patient transitioned to a second patient state based on at least one of the one or more biomarker signals; cause the stimulation generation circuitry to deliver electrical stimulation having a second parameter set based on the patient being in the second patient state; after the stimulation generation circuitry delivers electrical stimulation having the second parameter set, temporally cause the stimulation generation circuitry to cease delivery of electrical stimulation or to deliver electrical stimulation having the first parameter set; during the cessation of electrical stimulation or the stimulation generation circuitry delivering electrical stimulation having the first parameter set, determine whether the patient transitioned to the first patient state based on at least one of the one or more biomarker signals; and cause the stimulation generation circuitry to deliver electrical stimulation having the first parameter set or deliver electrical stimulation having the second parameter set based on the determination of whether the patient transitioned to the first patient state.

The details of one or more examples of the techniques of this disclosure are set forth in the accompanying drawings and the description below. Other features, objects, and advantages of the techniques will be apparent from the description and drawings, and from the claims.

### BRIEF DESCRIPTION OF DRAWINGS

FIG. 1 is a conceptual diagram illustrating an example system that includes an implantable medical device (IMD) configured to deliver electrical stimulation to a patient according to an example of the techniques of the disclosure.
FIG. 2 is a block diagram of the example IMD of FIG. 1 for delivering electrical stimulation according to an example of the techniques of the disclosure.
FIG. 3 is a block diagram of the external programmer of FIG. 1 for controlling delivery of electrical stimulation according to an example of the techniques of the disclosure.
FIG. 4 is a flowchart illustrating an example operation for selecting a therapy mode, in accordance with one or more examples described in this disclosure.
FIG. 5 is a flowchart illustrating another example operation for selecting a therapy mode, in accordance with one or more examples described in this disclosure.
FIG. 6 is a flowchart illustrating an example operation of therapy delivery in accordance with a first closed-loop therapy mode.
FIG. 7 is a flowchart illustrating an example operation of therapy delivery in accordance with a second closed-loop therapy mode.

### DETAILED DESCRIPTION

This disclosure describes example techniques for systems directed to optimizing ability to use physiological signals (e.g., biomarker signals) to inform (e.g., control) closed-loop therapy. In closed-loop therapy, a medical device senses biomarker signals (e.g., physiological signals generated by the patient's body and having one or more determined characteristics), and determines (e.g., sets or adjusts) therapy parameters (e.g., amplitude, pulse width, frequency, timing, etc.) based on the sensed biomarker signals. Particular signal characteristics (e.g., spectral characteristics) may indicate biomarkers. Accordingly, by detecting particular characteristics (like spectral power ratios for different bands or sub-bands, as one example) of physiological signals, a medical device may sense/detect biomarkers.

In some examples, based on the sensed biomarker signals, the medical device may adjust the therapy parameters of the therapy, and deliver therapy in accordance with the adjusted therapy parameters. However, in such cases, delivery of the therapy with the adjusted therapy parameters may result in confounding or suppressing the biomarker signals that were originally used for sensing. One example of confounding the biomarker signal is where the delivery of therapy interferes with the ability to sense the biomarker signals (e.g., the physiological electrical signal). One example of suppressing the biomarker signal is where the delivery of therapy causes an amplitude of the biomarker signal to be lowered to a level where the biomarker signal cannot be resolved. Stated another way, in some cases, the delivery of therapy confounds or suppresses the biomarker signals that were used as feedback to determine the therapy parameters for the therapy such that the biomarker signals cannot be sensed correctly, and creating difficulty in updating the therapy parameters.

In some cases, a patient exhibiting such behavior (e.g., where therapy confounds or suppresses the biomarker signals) may be deemed as not qualifying for closed-loop therapy, even though the patient may therapeutically benefit from closed-loop therapy. This disclosure describes example techniques to optimize ability to use closed-loop therapy even in cases where the delivery of therapy creates difficulty in accurately sensing the biomarker signals.

For instance, the biomarker signals may be indicative of a change in a patient state (e.g., from a first patient state to a second patient state). For example, the biomarker signals may be indicative of patient movement. There may be therapeutic benefit in delivering therapy having a first parameter set when the patient is in the first patient state, and delivering therapy having a second parameter set when the patient is in the second patient state. In this disclosure, a parameter set may refer to a set including amplitude, pulse width, and/or frequency. Also, in some examples, a particular parameter set may refer to no therapy being delivered (e.g., stimulation off), such as where the amplitude is zero.

The biomarker signals may be indicative of a patient transitioning from the first patient state to the second patient state, or vice-versa. As an example, when the patient is at rest, a brain signal may have a first amplitude, and when the patient is moving the brain signal may have a second amplitude. It may be desirable to provide no therapy when the patient is at rest, but provide therapy when the patient is moving. As another example, it may be desirable to provide therapy that assists with patient talking, when the patient is at rest, but provide therapy that assists with controlling patient movement (e.g., minimizing tremors) when the patient is moving.

However, for some patients, the delivery of therapy confounds (e.g., interferes with sensing of) or suppresses the biomarker signals used to determine patient state, making it difficult to accurately sense the biomarker signals. In such examples, it may be difficult for the medical device to utilize the biomarker signals to determine patient state, and thereby control the therapy that is to be delivered. For instance, for some patients, the delivery of therapy may confound or suppress the biomarker signals used to determine if the patient is moving or not. As an example of confounding, the delivery of therapy creates artifacts or saturation that makes it difficult to detect the biomarker in a signal. Therefore, for such a patient, once the patient starts to move, the medical device may not be able to determine, from the biomarker signals, that the patient stopped moving. Because the biomarker signals are confounded or suppressed, the medical device may not be able to adjust therapy based on the biomarker signals, and the patient may be deemed as not qualifying for normal closed-loop therapy. Again, the biomarker signal is an example of a signal that includes a biomarker, where the biomarker is a particular characteristic of the signal (e.g., spectral characteristic).

In accordance with one or more examples described in this disclosure, a medical device may be utilized to first determine if normal closed-loop therapy is available for a patient. Normal closed-loop therapy being available for the patient may mean that it is possible to determine based on the biomarker signal if the patient is changing states so that therapy can be adjusted when the patient changes states.

If normal closed-loop therapy is not available for a patient, in one or more examples, the medical device may be configured to deliver therapy in accordance with a polling closed-loop therapy. In the polling closed-loop therapy, the medical device may temporarily cease therapy delivery, allowing for the biomarker signals that were confounded or suppressed to return back to normal levels. The medical device may then sense the biomarker signals, and adjust therapy accordingly. Therefore, even though the biomarker signals in the patient may be suppressed or confounded by the therapy delivery, by temporally ceasing therapy and then sensing the biomarker signals, it may be possible to provide closed-loop therapy to the patient.

One example way in which to determine if normal closed-loop therapy is available is by the medical device delivering therapy to the patient in the first patient state (e.g., resting) and the second patient state (e.g., moving). In the presence of stimulation (e.g., in response to delivering the therapy) when the patient is in the first patient state, the medical device may sense the biomarker signal(s) (e.g., a first instance of a biomarker signal). In the presence of stimulation (e.g., in response to delivering the therapy) when the patient is in the second patient state, the medical device may sense the biomarker signals(s) (e.g., a second instance of the biomarker signal).

The medical device may determine whether there is a statistically significant difference between the first instance of the biomarker signal and the second instance of the biomarker signal (e.g., determine whether a difference between the first instance of the biomarker signal and the second instance of the biomarker signal satisfies a threshold). Examples of differences between the first instance and the second instance of the biomarker signals include differences in amplitude, differences in ratio of spectral powers in different frequency bands, differences in spectral powers in the same frequency band, etc. The medical device may select a therapy mode based on whether there is a statistically significant difference between the first instance of the biomarker signal and the second instance of the biomarker signal.

For instance, if there is a statistically significant difference, the medical device may determine to deliver therapy in accordance with a first closed-loop therapy mode (e.g., a normal closed-loop therapy mode). Because there is a statistically significant difference between the first instance of the biomarker signal and the second instance of the biomarker signal, the medical device may be able to determine whether the patient transitions from the first patient state to the second patient state based on the biomarker signal(s), and vice-versa. Therefore, normal closed-loop therapy mode therapy may provide therapeutic relief to the patient.

If there is not a statistically significant difference, the medical device may determine to deliver therapy in accordance with a second closed-loop therapy mode that is different than the first closed-loop therapy mode (e.g., a polling closed-loop therapy mode). Because there is not a statistically significant difference between the first instance of the biomarker signal and the second instance of the biomarker signal, the medical device may not be able to determine whether the patient transitions from the first patient state to the second patient state based on the biomarker signal(s), and vice-versa. Therefore, polling closed-loop therapy mode therapy may provide therapeutic relief to the patient where therapy is temporally ceased to allow the sensed biomarker signal(s) to return to normal levels or not be confounded by the therapy.

The above describes examples of patient transitioning from a first patient state to a second patient state and back (e.g., not moving, to moving, and then back to not moving). However, the example techniques are not so limited. The example techniques may be applicable to examples where the patient transitions between more than two states. For instance, with respect to medication, a first patient state may be when the patient takes medication, the second patient state may be when the medication is effective, and the third patient state may be when the medication is no longer effective. In some examples, the second patient state may be considered as one of when the medication is effective and when the medication is no longer effective. In accordance with one or more examples, there may be different stimulation sets that are delivered based on whether the patient is in the first, second, or third patient states.

As another example, a first patient state may be when the patient is awake and standing, and a second patient state may be when the patient is asleep and lying down. In some examples, there may be a third patient state when patient is lying down awake and before falling asleep. In such examples, there may be stimulation parameter set that is delivered based on whether the patient is in the first, second, or third patient states. In general, the example techniques may be applicable to N patient states. In some examples, the patient states may include a plurality of factors. For instance, a first patient state may be when the patient has just taken medication and is not moving, a second patient state may be when the patient has just taken medication and is moving, and so forth. The techniques of this disclosure should not be considered limited to the examples provided to assist with understanding.

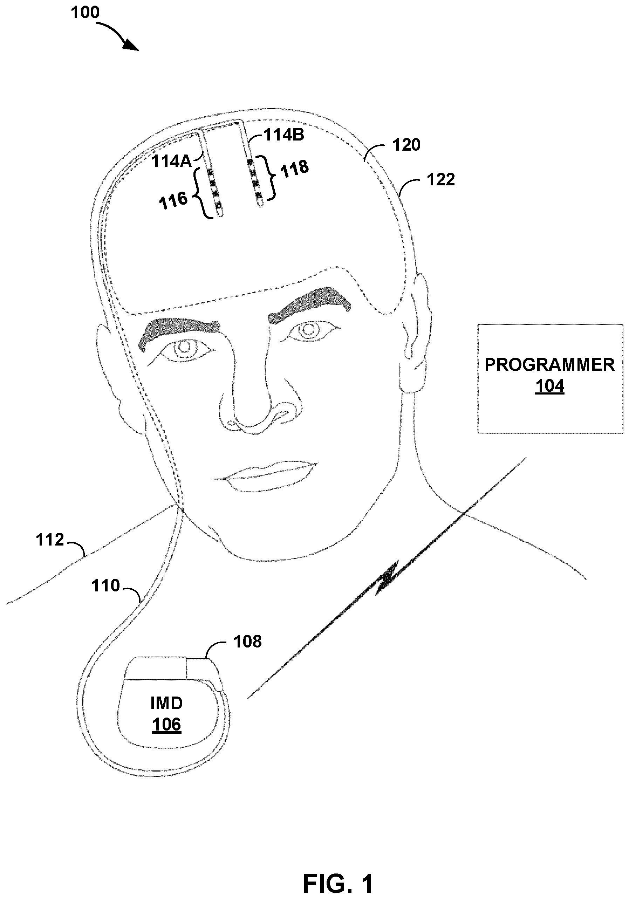

FIG. 1 is a conceptual diagram illustrating an example system 100 that includes an implantable medical device (IMD) 106 configured to deliver adaptive deep brain stimulation (DBS) to a patient 112. DBS may be adaptive in the sense that IMD 106 may adjust, increase, or decrease the magnitude of one or more parameters of the DBS in response to changes in patient activity or movement, a severity of one or more symptoms of a disease of the patient, a presence of one or more side effects due to the DBS, or one or more sensed signals of the patient.

For instance, one example of system 100 is a DBS system with capabilities to both deliver stimulation and sense intrinsic neuronal signals, also referred to as biomarker signals, and determine (e.g., detect) biomarkers from the biomarker signals. System 100 may provide for "closed-loop" therapy where IMD 106 may continuously monitor the state of certain biomarker signals and deliver stimulation according to pre-programmed routines based on the biomarker signals.

System 100 may be configured to treat a patient condition, such as a movement disorder, neurodegenerative impairment, a mood disorder, or a seizure disorder of patient 112. Patient 112 ordinarily is a human patient. In some cases, however, therapy system 100 may be applied to other mammalian or non-mammalian, non-human patients. While movement disorders and neurodegenerative impairment are primarily referred to herein, in other examples, therapy system 100 may provide therapy to manage symptoms of other patient conditions, such as, but not limited to, essential tremors (ETs), seizure disorders (e.g., epilepsy) or mood (or psychological) disorders (e.g., major depressive disorder (MDD), Parkinson's disease, bipolar disorder, anxiety disorders, post-traumatic stress disorder, dysthymic disorder, and obsessive-compulsive disorder (OCD)). At least some of these disorders may be manifested in one or more patient movement behaviors. As described herein, a movement disorder or other neurodegenerative impairment may include symptoms such as, for example, muscle control impairment, motion impairment or other movement problems, such as rigidity, spasticity, bradykinesia, rhythmic hyperkinesia, nonrhythmic hyperkinesia, and akinesia. In some cases, the movement disorder may be a symptom of Parkinson's disease. However, the movement disorder may be attributable to other patient conditions.

Example therapy system 100 includes medical device programmer 104, implantable medical device (IMD) 106, lead extension 110, and leads 114A and 114B with respective sets of electrodes 116, 118. In the example shown in FIG. 1, electrodes 116, 118 of leads 114A, 114B are positioned to deliver electrical stimulation to a tissue site within brain 120, such as a deep brain site under the dura mater of brain 120 of patient 112. In some examples, delivery of stimulation to one or more regions of brain 120, such as the subthalamic nucleus, globus pallidus or thalamus, may be an effective treatment to manage movement disorders, such as Parkinson's disease. Some or all of electrodes 116, 118 also may be positioned to sense neurological brain signals within brain 120 of patient 112. In some examples, some of electrodes 116, 118 may be configured to sense neurological brain signals and others of electrodes 116, 118 may be configured to deliver adaptive electrical stimulation to brain 120. In other examples, all of electrodes 116, 118 are configured to both sense neurological brain signals (e.g., biomarker signals) and deliver adaptive electrical stimulation to brain 120. In some examples, unipolar stimulation may be possible where one electrode is on the housing of IMD 106.

IMD 106 includes a therapy module (e.g., which may include processing circuitry, stimulation generation circuitry, sensing circuitry, or other electrical circuitry configured to perform the functions attributed to IMD 106) that includes a stimulation generator configured to generate and deliver electrical stimulation therapy to patient 112 via a subset of electrodes 116, 118 of leads 114A and 114B, respectively. The subset of electrodes 116, 118 that are used to deliver electrical stimulation to patient 112, and, in some cases, the polarity of the subset of electrodes 116, 118, may be referred to as a stimulation electrode combination. As described in further detail below, the stimulation electrode combination can be selected for a particular patient 112 and target tissue site (e.g., selected based on the patient condition). The group of electrodes 116, 118 includes at least one electrode and can include a plurality of electrodes. In some examples, the plurality of electrodes 116 and/or 118 may have a complex electrode geometry such that two or more electrodes are located at different positions around the perimeter of the respective lead.

In some examples, the biomarker signals sensed within brain 120 may reflect changes in electrical current produced by the sum of electrical potential differences across brain tissue. Examples of neurological brain signals include, but are not limited to, bioelectric signals generated from local field potentials (LFP) sensed within one or more regions of brain 120. Electroencephalogram (EEG) signal or an electrocorticogram (ECoG) signal are also examples of bioelectric signals. For example, neurons generate the bioelectric signals, and if measured at depth, it is LFP, if measured on the coretex, it is ECoG, and if on scalp, it is EEG.

One example of the feature of interest (e.g., biomarker) within the LFPs (e.g., a biomarker signal) is synchronized beta frequency band (13-33Hz) LFP activity recorded within the sensorimotor region of the subthalamic nucleus (STN) in Parkinson's disease patients. The source of the LFP activity can be considered as an oscillatory signal source, within the brain of the patient, that outputs an oscillatory electrical voltage signal that is sensed by one or more of electrodes 116 and/or 118. The suppression of pathological beta activity (e.g., suppression or squelching of the signal component of the bioelectric signals generated from the oscillatory LFP signal source that is within the beta frequency band) by both medication and DBS may correlate with improvements in the motor symptoms of patients who have Parkinson's disease.

In some examples, the neurological brain signals that are used to select a stimulation electrode combination may be sensed within the same region of brain 120 as the target tissue site for the electrical stimulation. As previously indicated, these tissue sites may include tissue sites within anatomical structures such as the thalamus, subthalamic nucleus or globus pallidus of brain 120, as well as other target tissue sites. The specific target tissue sites and/or regions within brain 120 may be selected based on the patient condition. Thus, in some examples, both stimulation electrode combinations and sense electrode combinations may be selected from the same set of electrodes 116, 118. In other examples, the electrodes used for delivering electrical stimulation may be different than the electrodes used for sensing neurological brain signals.

Electrical stimulation generated by IMD 106 may be configured to manage a variety of disorders and conditions. In some examples, the stimulation generator of IMD 106 is configured to generate and deliver electrical stimulation pulses to patient 112 via electrodes of a selected stimulation electrode combination. However, in other examples, the stimulation generator of IMD 106 may be configured to generate and deliver a continuous wave signal, e.g., a sine wave or triangle wave. In either case, a stimulation generator within IMD 106 may generate the electrical stimulation therapy for DBS according to a selected therapy program. In examples in which IMD 106 delivers electrical stimulation in the form of stimulation pulses, a therapy program may include a set of therapy parameter values (e.g., stimulation parameters), such as a stimulation electrode combination for delivering stimulation to patient 112, pulse frequency, pulse width, and a current or voltage amplitude of the pulses. As previously indicated, the electrode combination may indicate the specific electrodes 116, 118 that are selected to deliver stimulation signals to tissue of patient 112 and the respective polarities of the selected electrodes. The electrical stimulation generated by IMD 106 may generate, for example, burst pulses, interleaved pulses, or concurrent pulses.

In some examples, electrodes 116, 118 may be radially-segmented DBS arrays (rDBSA) of electrodes. Radially-segmented DBS arrays refer to electrodes that are segmented radially along the lead. As one example, leads 114A and 114B may include a first set of electrodes arranged circumferentially around leads 114A and 114B that are all at the same height level on leads 114A and 114B. Each of the electrodes in the first set of electrodes is a separate segmented electrode and form a level of radially-segmented array of electrodes. Leads 114A and 114B may include a second set of electrodes arranged circumferentially around leads 114A and 114B that are all at the same height level on leads 114A and 114B. Each of the electrodes in the first set of electrodes is a separate segmented electrode and form a level of radially-segmented array of electrodes. The rDBSA electrodes may be beneficial for directional stimulation and/or sensing.

The signal component in the beta frequency band is described as one example, and the techniques are applicable to other types of LFP activity. Furthermore, the example techniques are not limited to examples where electrodes 116, 118 are an rDBSA of electrodes. The example of using an rDBSA of electrodes is described as a way of directional stimulation and sensing. However, the example techniques are also useable in examples where directional stimulation and sensing are not available or are not used. Moreover, there may be other ways of performing directional stimulation and sensing that do not require the use of an rDBSA of electrodes.

To suppress the signal component having the beta frequency band from the oscillatory signal source, IMD 106 may output an electrical stimulation signal that alters the way in which neurons of the oscillatory signal source produce signals. For example, the electrical stimulation either directly inhibits a certain neuronal population that includes the oscillatory signal source or excites one group of neurons which in turn suppresses another group of neurons (e.g., network effect). The stimulation may act on the neurons directly, and not necessarily on the signals the neurons (e.g., oscillatory signal source) produces.

In the above example, IMD 106 may output the electrical stimulation signal to suppress neurons. However, the example techniques are not so limited. IMD 106 may be configured to deliver electrical stimulation that provides therapeutic effect without necessarily needing to suppress neurons.

IMD 106 may be implanted within a subcutaneous pocket above the clavicle, or, alternatively, on or within cranium 122 or at any other suitable site within patient 112. Generally, IMD 106 is constructed of a biocompatible material that resists corrosion and degradation from bodily fluids. IMD 106 may comprise a hermetic housing to substantially enclose components, such as a processor, therapy module, and memory.

As shown in FIG. 1, implanted lead extension 110 is coupled to IMD 106 via connector 108 (also referred to as a connector block or a header of IMD 106). In the example of FIG. 1, lead extension 110 traverses from the implant site of IMD 106 and along the neck of patient 112 to cranium 122 of patient 112 to access brain 120. In the example shown in FIG. 1, leads 114A and 114B (collectively "leads 114") are implanted within the right and left hemispheres (or in just one hemisphere in some examples), respectively, of patient 112 in order to deliver electrical stimulation to one or more regions of brain 120, which may be selected based on the patient condition or disorder controlled by therapy system 100. The specific target tissue site and the stimulation electrodes used to deliver stimulation to the target tissue site, however, may be selected, e.g., according to the identified patient behaviors and/or other sensed patient parameters. For example, the target tissue site may be the location of the oscillatory signal source that generates the bioelectric signal having a signal component in the beta frequency band. The stimulation electrodes used to deliver stimulation to the target tissue site may be those that are most proximal to the oscillatory signal source, e.g., using the example techniques described in this disclosure. Other lead 114 and IMD 106 implant sites are contemplated. For example, IMD 106 may be implanted on or within cranium 122, in some examples. Leads 114A and 114B may be implanted within the same hemisphere or IMD 106 may be coupled to a single lead implanted in a single hemisphere, in some examples.

Existing lead sets include axial leads carrying ring electrodes disposed at different axial positions and so-called "paddle" leads carrying planar arrays of electrodes. Selection of electrode combinations within an axial lead, a paddle lead, or among two or more different leads presents a challenge to the clinician. In some examples, more complex lead array geometries may be used.

Although leads 114 are shown in FIG. 1 as being coupled to a common lead extension 110, in other examples, leads 114 may be coupled to IMD 106 via separate lead extensions or directly to connector 108. Leads 114 may be positioned to deliver electrical stimulation to one or more target tissue sites within brain 120 to manage patient symptoms associated with a movement disorder of patient 112. Leads 114 may be implanted to position electrodes 116, 118 at desired locations of brain 120 through respective holes in cranium 122. Leads 114 may be placed at any location within brain 120 such that electrodes 116, 118 are capable of providing electrical stimulation to target tissue sites within brain 120 during treatment. For example, electrodes 116, 118 may be surgically implanted under the dura mater of brain 120 or within the cerebral cortex of brain 120 via a burr hole in cranium 122 of patient 112, and electrically coupled to IMD 106 via one or more leads 114.

In the example shown in FIG. 1, electrodes 116, 118 of leads 114 are shown as ring electrodes. Ring electrodes may be used in DBS applications because ring electrodes are relatively simple to program and are capable of delivering an electrical field to any tissue adjacent to electrodes 116, 118. In other examples, electrodes 116, 118 may have different configurations. For example, at least some of the electrodes 116, 118 of leads 114 may have a complex electrode array geometry that is capable of producing shaped electrical fields. The complex electrode array geometry may include multiple electrodes (e.g., partial ring or segmented electrodes, as mentioned above) around the outer perimeter of each lead 114, rather than one ring electrode. In this manner, electrical stimulation may be directed in a specific direction from leads 114 to enhance therapy efficacy and reduce possible adverse side effects from stimulating a large volume of tissue.

In some examples, a housing of IMD 106 may include one or more stimulation and/or sensing electrodes. In some examples, leads 114 may have shapes other than elongated cylinders as shown in FIG. 1. For example, leads 114 may be paddle leads, spherical leads, bendable leads, or any other type of shape effective in treating patient 112 and/or minimizing invasiveness of leads 114.

IMD 106 includes a memory to store a plurality of therapy programs that each define a set of therapy parameter values. In some examples, IMD 106 may select a therapy program from the memory based on various parameters, such as sensed patient parameters and the identified patient behaviors. IMD 106 may generate electrical stimulation based on the parameters of the selected therapy program to manage the patient symptoms associated with a movement disorder.

External programmer 104 wirelessly communicates with IMD 106 as needed to provide or retrieve therapy information. Programmer 104 is an external computing device that the user, e.g., a clinician and/or patient 112, may use to communicate with IMD 106. For example, programmer 104 may be a clinician programmer that the clinician uses to communicate with IMD 106 and program one or more therapy programs for IMD 106. Alternatively, programmer 104 may be a patient programmer that allows patient 112 to select programs and/or view and modify therapy parameters. The clinician programmer may include more programming features than the patient programmer. In other words, more complex or sensitive tasks may only be allowed by the clinician programmer to prevent an untrained patient from making undesirable changes to IMD 106.

When programmer 104 is configured for use by the clinician, programmer 104 may be used to transmit initial programming information to IMD 106. This initial information may include hardware information, such as the type of leads 114 and the electrode arrangement, the position of leads 114 within brain 120, the configuration of electrode array 116, 118, initial programs defining therapy parameter values, and any other information the clinician desires to program into IMD 106. Programmer 104 may also be capable of completing functional tests (e.g., measuring the impedance of electrodes 116, 118 of leads 114).

The clinician may also store therapy programs within IMD 106 with the aid of programmer 104. During a programming session, the clinician may determine one or more therapy programs that may provide efficacious therapy to patient 112 to address symptoms associated with the patient condition, and, in some cases, specific to one or more different patient states, such as a sleep state, movement state or rest state. For example, the clinician may select one or more stimulation electrode combinations with which stimulation is delivered to brain 120. During the programming session, the clinician may evaluate the efficacy of the specific program being evaluated based on feedback provided by patient 112 or based on one or more physiological parameters of patient 112 (e.g., muscle activity, muscle tone, rigidity, tremor, etc.). Alternatively, identified patient behavior from video information may be used as feedback during the initial and subsequent programming sessions. Programmer 104 may assist the clinician in the creation/identification of therapy programs by providing a methodical system for identifying potentially beneficial therapy parameter values.

However, in some examples, IMD 106 or programmer 104 (e.g., a medical device), alone or in combination, may automatically determine electrode configuration and therapy parameters. For example, the medical device may determine which electrodes to use for stimulation based on which electrodes are most proximal to the oscillatory signal source. In some examples, programmer 104 may output information indicating the selected electrode configuration for stimulation and the determined stimulation amplitude or other therapy parameter for the clinician or physician to review and confirm before IMD 106 delivers therapy via the selected electrode configuration with the determined stimulation amplitude.

Programmer 104 may also be configured for use by patient 112. When configured as a patient programmer, programmer 104 may have limited functionality (compared to a clinician programmer) in order to prevent patient 112 from altering critical functions of IMD 106 or applications that may be detrimental to patient 112. In this manner, programmer 104 may only allow patient 112 to adjust values for certain therapy parameters or set an available range of values for a particular therapy parameter.

Programmer 104 may also provide an indication to patient 112 when therapy is being delivered, when patient input has triggered a change in therapy or when the power source within programmer 104 or IMD 106 needs to be replaced or recharged. For example, programmer 104 may include an alert LED, may flash a message to patient 112 via a programmer display, generate an audible sound or somatosensory cue to confirm patient input was received, e.g., to indicate a patient state or to manually modify a therapy parameter.

Therapy system 100 may be implemented to provide chronic stimulation therapy to patient 112 over the course of several months or years. However, system 100 may also be employed on a trial basis to evaluate therapy before committing to full implantation. If implemented temporarily, some components of system 100 may not be implanted within patient 112. For example, patient 112 may be fitted with an external medical device, such as a trial stimulator, rather than IMD 106. The external medical device may be coupled to percutaneous leads or to implanted leads via a percutaneous extension. If the trial stimulator indicates DBS system 100 provides effective treatment to patient 112, the clinician may implant a chronic stimulator within patient 112 for relatively long-term treatment.

Although IMD 106 is described as delivering electrical stimulation therapy to brain 120, IMD 106 may be configured to direct electrical stimulation to other anatomical regions of patient 112. Further, an IMD may provide other electrical stimulation such as spinal cord stimulation to treat a movement disorder. For instance, the example of DBS therapy is provided for ease of illustration. In general, the example techniques described in this disclosure may be utilized for various therapies such as stimulation of spinal cord for pain therapy or nerves attached to the spinal cord such as sacral nerves, tibial nerves, etc. Also, the neural signals generated within the brain are one example of biomarker signals, and there may be other examples of biomarker signals such as evoked compound action potentials (ECAPs) or electromyography (EMG) signals.

As described above, IMD 106 uses biomarker signals (e.g., physiological signals generated by the body of patient 112 like LFPs) as feedback to determine the therapy parameters of the therapy that is to be provided. In some examples, IMD 106 may utilize the biomarker signals as a way to determine a change of patient state. For instance, a biomarker signal may indicate when patient 112 is moving. Examples of the biomarker signal includes signals in the beta band (e.g., 13-38 Hz), such as movement-related beta desynchronization (MRBD), signals in the gamma band (e.g., 39-42 Hz), such as dyskinesia markers, signals in the theta band (e.g., 4-7 Hz), such as signals indicative of tremor, or signals in the delta band (e.g., 1-3 Hz), such as signals indicative of sleep. IMD 106 may be configured to deliver therapy having different parameters based on whether patient 112 is moving or not.

As an example, when patient 112 is in a first patient state (e.g., not moving), IMD 106 may be configured to deliver electrical stimulation having a first parameter set. When patient 112 is in a second patient state (e.g., moving), IMD 106 may be configured to deliver electrical stimulation having a second parameter set. The first parameter set and the second parameter set may each include a set of respective amplitudes, pulse widths, frequencies, etc. In some examples, the first parameter set and/or the second parameter set include examples where the first parameter set and/or the second parameter set refer to "stimulation off" (e.g., amplitude is zero).

For instance, when patient 112 is in the first patient state (e.g., not moving), IMD 106 may be configured to deliver no stimulation (e.g., stimulation off). In this example, the electrical stimulation having a first parameter set refers to no stimulation. As another example, when patient 112 is in the first patient state (e.g., not moving), IMD 106 may be configured to deliver stimulation that assists with talking. In this example, the electrical stimulation having a first parameter set refers to stimulation having amplitude, pulse width, and/or frequency that assists with patient talking.

IMD 106 may utilize the biomarker signals to determine whether patient 112 transitions from one patient state to another. However, there is a possibility that the delivery of the electrical stimulation confounds (e.g., interferes with the sensing of) or suppresses the biomarker signals that are used to determine change in patient state. As one example, the delivery of the electrical stimulation signal may suppress the biomarker signal that are used to determine the patient state. For instance, the delivery of electrical stimulation may cause an amplitude or other characteristic of the biomarker signal to be reduced to a level where the biomarker signal cannot be accurately sensed.

As another example, the delivery of electrical stimulation signal may interfere with the sensing of the biomarker signal, as an example of the electrical stimulation confounding the biomarker signal. For instance, if the frequency of the electrical stimulation signal is in the same band as the biomarker signal that is to be sensed to determine change in patient state, then there is a possibility that IMD 106 may not be able to resolve whether the sensed signal is the actual biomarker signal or whether the sensed signal is an artifact of the electrical stimulation signal. For instance, the electrical stimulation may cause artifacts or saturate the sensing so that IMD 106 may not be able to differentiate between the biomarker signals and the stimulation.

In cases where the delivery of electrical stimulation confounds or suppresses the biomarker signal used for normal closed-loop therapy, normal closed-loop therapy may be turned off for patient 112. For instance, if the delivery of electrical stimulation confounds or suppresses the biomarker signal used for normal closed-loop therapy, then IMD 106 may not be able to determine whether patient 112 changed patient states or not when therapy is being delivered.

As an illustrative example, the biomarker signal may indicate if patient 112 is moving or not. IMD 106 may deliver electrical stimulation when patient 112 is moving, and turn off stimulation when patient 112 is not moving. In such cases, when patient 112 is moving, IMD 106 may deliver electrical stimulation. However, when patient 112 stops moving, IMD 106 may not be able to determine that patient 112 stopped moving because the electrical stimulation may confound or suppress the biomarker signal that IMD 106 uses to determine that patient 112 stopped moving. In this case, IMD 106 may keep delivering electrical stimulation even though patient 112 stopped moving, resulting in continuous therapy instead of closed-loop therapy.

This disclosure describes example techniques to determine if normal closed-loop therapy can be implemented for a given patient at current settings and biomarker signals. As also described in this disclosure, if current settings and biomarker signals are insufficient for normal closed-loop therapy, this disclosure describes example techniques to configure IMD 106 in a way that achieves the benefits of closed-loop therapy.

In accordance with one or more examples described in this disclosure, during surgery, shortly after surgery for implantation of IMD 106, or possibly even after patient 112 has been receiving therapy for a while and would like to update therapy, a medical practitioner (e.g., the surgeon or clinician) may request patient 112 to be in a first patient state. The medical practitioner may cause IMD 106 to deliver electrical stimulation when patient 112 is in the first patient state, and sense a first instance of a biomarker signal in the presence of electrical stimulation (e.g., after or in response to the delivery of electrical stimulation). The medical practitioner may ask patient 112 to be in a second patient state. The medical practitioner may cause IMD 106 to deliver electrical stimulation when patient 112 is in the second patient state, and sense a second instance of the same biomarker signal in the presence of electrical stimulation (e.g., after or in response to the delivery of electrical stimulation).

If there is a statistically significant difference between the first instance of the biomarker signal and the second instance of the biomarker signal, then IMD 106 may be able to determine a change in patient state based on the biomarker signal. That is, the delivery of the electrical stimulation is not overly confounding or suppressing the biomarker signal, and even with electrical stimulation, IMD 106 is able to determine a change in patient state based on the biomarker signal. In such cases, normal closed-loop therapy may work well for patient 112.

For instance, in normal closed-loop therapy, IMD 106 may be configured to deliver electrical stimulation having a first parameter set based on patient 112 being in the first patient state, and deliver electrical stimulation having a second parameter set based on patient 112 being in a second patient state. IMD 106 may transition between delivering electrical having the first parameter set based on the patient being in the first patient state and delivering electrical stimulation having the second parameter set based on the patient being in the second patient state. IMD 106 may determine when patient 112 transitioned from the first patient state to the second patient state, and vice-versa based on the sensed biomarker signal.

However, if there is not a statistically significant difference between the first instance of the biomarker signal and the second instance of the biomarker signal, then IMD 106 may not be able to use the biomarker signal to determine when patient 112 changes patient states, and thereby not determine whether to change the electrical stimulation. This disclosure describes examples of closed-loop therapy, referred to as polling closed-loop therapy, in which IMD 106 may still be able to achieve some of the benefits of normal closed-loop therapy.

In the polling closed-loop therapy, IMD 106 may temporally cease delivery of the electrical stimulation therapy that confounds or suppresses the biomarker signal used to determine patient state. Once the delivery of electrical stimulation therapy is ceased, the biomarker signal that was being confounded or suppressed by the electrical stimulation can be properly sensed, and IMD 106 can determine the patient state based on the biomarker signal. If there is a change in patient state, then IMD 106 may change the electrical stimulation parameters. If there is no change in patient state, then IMD 106 may restart the electrical stimulation in accordance with the electrical stimulation parameters.

The amount of time that the stimulation is ceased may be less than a second, less than 500 milliseconds, less than 1 millisecond, less than 500 microseconds, or less than 100 microseconds. In some examples, the amount of time that the stimulation is ceased may be based on the patient condition. For example, for a first patient condition, the stimulation may be ceased for 500 milliseconds, but for a second patient condition, the stimulation may be ceased for 100 microseconds. The amount of time that the stimulation may be ceased may be based on "washout" period that a biomarker signal presents. The washout period may be the amount of time that the effects of the stimulation persist even when delivery is stopped. In some examples, the stimulation may be ceased for minutes.

Assume that IMD 106 is configured to deliver electrical stimulation having a first parameter set when patient 112 is in a first patient state, and configured to deliver electrical stimulation having a second parameter set when patient 112 is in a second patient state. Also, assume that delivery of electrical stimulation having the first parameter set does not confound or suppress the biomarker signal used to determine change in patient state, but delivery of electrical stimulation having the second parameter set does confound or suppress the biomarker signal used to determine change in patient state. Accordingly, in this example, when patient 112 is in the first patient state with delivery of electrical stimulation having the first parameter set, IMD 106 may be able to determine when patient 112 transitions from the first patient state to the second patient state (again, in this example, electrical stimulation having the first parameter set does not confound or suppressed the biomarker signal).

When patient 112 transitions to the second patient state, IMD 106 may deliver electrical stimulation having the second parameter set. However, IMD 106 may not be able to determine when patient 112 transitions from the second patient state to the first patient state because electrical stimulation having the second parameter set may confound or suppress the biomarker signal used to determine patient state. In such cases, IMD 106 may cease to temporally deliver electrical stimulation having the second parameter set or deliver electrical stimulation having the first parameter set. In this case, IMD 106 may be able to properly determine the biomarker signal that was confounded or suppressed, and determine if patient 112 changed patient states from the second patient state back to the first patient state.

If patient 112 changed patient states from the second patient state back to the first patient state, then IMD 106 may deliver electrical stimulation having the first parameter set. However, if patient 112 did not change states from the second patient state back to the first patient state, then IMD 106 may resume delivering electrical stimulation having the second parameter set.

In this way, even though normal closed-loop therapy may not be available, polling closed-loop therapy may still provide some of the benefits of normal closed-loop therapy. For instance, the amount of time that electrical stimulation is ceased may be a relatively small amount of time (e.g., less than one second, less than 500 milliseconds, less than 100 milliseconds, less than 500 micro-seconds, and less than 100 micro-seconds). In this short amount of time, the biomarker signals may resume and may be sensed properly, but the amount of time may be short enough that patient 112 may not experience negative side effects or undesirable return of symptoms.

In the above example, if normal closed-loop therapy is unavailable, IMD 106 may deliver therapy in accordance with polling closed-loop therapy. In some examples, the medical practitioner may first confirm that normal closed-loop therapy is unavailable. For example, the medical practitioner may reduce the stimulation intensity, and determine if normal closed-loop therapy is available at a lower stimulation intensity that still provides therapeutic effect. If there is no stimulation intensity that provides therapeutic effect, and where normal closed-loop therapy is available, IMD 106 may deliver electrical stimulation in accordance with polling closed-loop therapy.

The above examples are described with respect to a first patient state (e.g., not moving) and a second patient state (e.g., moving). However, there may be other types of patient states such as whether the patient has taken medication or not. For instance, the first patient state may be with the patient having taken medication, and the second patient state may be one of the steady state of the medication or when medication is no longer effective. As another example, the first patient state may be when the patient is awake and standing, and the second patient state may be when then patient is lying down and asleep. There may also be more than two patient states (e.g., N number of patient states). There may be parameter sets associated with each of the patient states. In accordance with one or more examples described in this disclosure, IMD 106 may utilize the example techniques described in this disclosure to determine whether the patient transitioned from one patient state to another, regardless of the number of patient states that may be present.

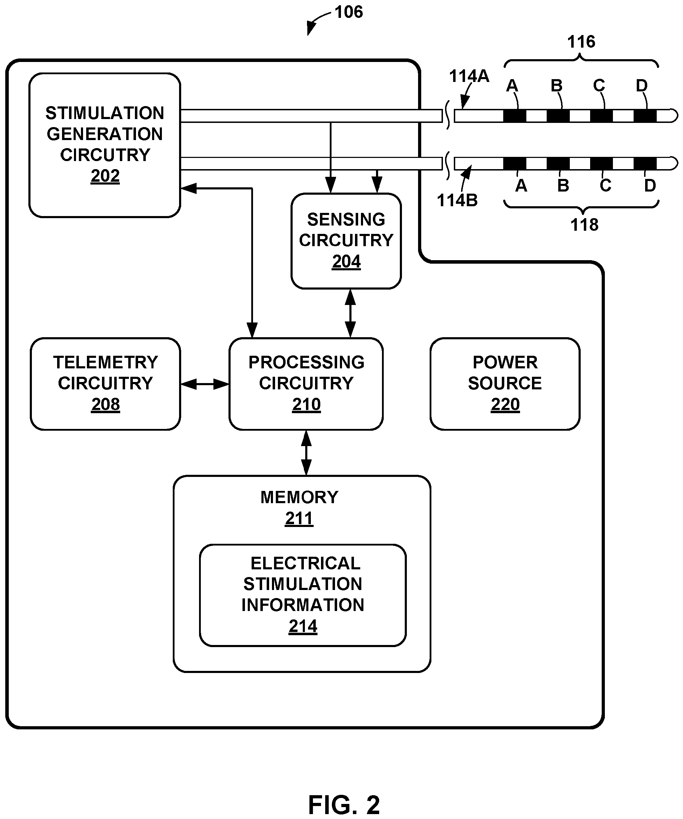

FIG. 2 is a block diagram of the example IMD 106 of FIG. 1 for delivering adaptive deep brain stimulation therapy. In the example shown in FIG. 2, IMD 106 includes processing circuitry 210, memory 211, stimulation generation circuitry 202, sensing circuitry 204, and telemetry circuitry 208, and power source 220. Each of these circuits may be or include electrical circuitry configured to perform the functions attributed to each respective circuit. Memory 211 may include any volatile or non-volatile media, such as a random-access memory (RAM), read only memory (ROM), non-volatile RAM (NVRAM), electrically erasable programmable ROM (EEPROM), flash memory, and the like. Memory 211 may store computer-readable instructions that, when executed by processing circuitry 210, cause IMD 106 to perform various functions. Memory 211 may be a storage device or other non-transitory medium.

In the example shown in FIG. 2, memory 211 stores electrical stimulation information 214. Electrical stimulation information 214 may include program parameters (e.g., a therapy parameter set), such as a stimulation electrode combination, electrode polarity, current or voltage amplitude, pulse width, and pulse rate. In some examples, individual therapy programs may be stored as a therapy group, which defines a set of therapy programs with which stimulation may be generated. The stimulation signals defined by the therapy programs of the therapy group may be delivered together on an overlapping or non-overlapping (e.g., time-interleaved) basis.

Stimulation generation circuitry 202, under the control of processing circuitry 210, generates stimulation signals for delivery to patient 112 via selected combinations of electrodes 116, 118. An example range of electrical stimulation parameters believed to be effective in DBS to manage a movement disorder of patient include:
1. Pulse Rate, i.e., Frequency: between approximately 40 Hertz and approximately 500 Hertz, such as between approximately 90 to 170 Hertz or such as approximately 90 Hertz.
2. In the case of a voltage controlled system, Voltage Amplitude: between approximately 0.1 volts and approximately 50 volts, such as between approximately 2 volts and approximately 3 volts.
3. In the case of a current controlled system, Current Amplitude: between approximately 1 milliamps to approximately 3.5 milliamps, such as between approximately 1.0 milliamps and approximately 1.75 milliamps.
4. Pulse Width: between approximately 50 microseconds and approximately 500 microseconds, such as between approximately 50 microseconds and approximately 200 microseconds.

Accordingly, in some examples, stimulation generation circuitry 202 may generate electrical stimulation signals in accordance with the electrical stimulation parameters noted above. Other ranges of therapy parameter values may also be useful, and may depend on the target stimulation site within patient 112. While stimulation pulses are described, stimulation signals may be of any form, such as continuous-time signals (e.g., sine waves) or the like.

As one example, for processing circuitry 210 to determine whether normal closed-loop therapy or polling closed-loop therapy should be used for patient 112, stimulation generation circuitry 202 may deliver electrical stimulation with patient 112 in a first patient state, and deliver electrical stimulation with patient 112 in a second patient state. For each instance that stimulation generation circuitry 202 delivered electrical stimulation, processing circuitry 210 may receive respective first and second instances of a biomarker signal. Based on whether a difference between the first and second instances of the biomarker signal is statistically significant (e.g., difference between the first and second instances of the biomarker signal satisfies a threshold), processing circuitry 210 may select a therapy mode (e.g., first closed-loop therapy mode or second closed-loop therapy mode). One example of the first closed-loop therapy mode is a normal closed-loop therapy mode. One example of the second closed-loop therapy mode is a polling closed-loop therapy mode.

Stimulation generation circuitry 202 may then deliver electrical stimulation in accordance with the selected therapy mode. For example, electrical stimulation information 214 may include information for a first set of therapy parameters and a second set of therapy parameters. Stimulation generation circuitry 202 may deliver electrical stimulation having the first set of therapy parameters when patient 112 is in a first patient state, and deliver stimulation having the second set of therapy parameters with patient 112 is in a second patient state.

Processing circuitry 210 may be configured to determine whether patient 112 is in the first patient state or the second patient state based on one or more biomarker signals sensed by sensing circuitry 204. Although sensing circuitry 204 is incorporated into a common housing with stimulation generation circuitry 202 and processing circuitry 210 in FIG. 2, in other examples, sensing circuitry 204 may be in a separate housing from IMD 106 and may communicate with processing circuitry 210 via wired or wireless communication techniques. Example neurological brain signals include, but are not limited to, a signal generated from local field potentials (LFPs) within one or more regions of brain 120. EEG and ECoG signals are examples of local field potentials that may be measured within brain 120. LFPs, EEG and ECoG may be different measurements of the same bioelectric signals in the brain. The neurons generate the signals, and if measured at depth, it is LFP, if measured on the coretex, it is ECoG, and if on the scalp, it is EEG. In general, the bioelectric marker signals may be formed by one or more oscillatory signal sources. The set of electrodes 116 and 118 that are most proximate to the oscillatory signal sources are good candidates to use for delivering therapy.

Processing circuitry 210 of IMD 106 may sense, via electrodes 116, 118 interposed along leads 114 (and sensing circuitry 204), one or more biomarker signals of brain 120 of patient 112. Further, processing circuitry 210 of IMD 106 may deliver, via electrodes 116, 118 (and stimulation generation circuitry 202), electrical stimulation therapy to patient 112 based on the sensed one or more biomarker signals of brain 120. The adaptive DBS therapy is defined by electrical stimulation information 214. For example, electrical stimulation information 214 may include a current amplitude (for a current-controlled system) or a voltage amplitude (for a voltage-controlled system), a pulse rate or frequency, and a pulse width, or a number of pulses per cycle. In examples where the electrical stimulation is delivered according to a "burst" of pulses, or a series of electrical pulses defined by an "on-time" and an "off-time," the one or more parameters may further define one or more of a number of pulses per burst, an on-time, and an off time. Processing circuitry 210, via electrodes 116, 118, delivers to patient 112 adaptive DBS and may adjust one or more parameters defining the electrical stimulation based on corresponding parameters of the sensed one or more bioelectric signals of brain 120.

In some examples, processing circuitry 210 may continuously measure the one or more bioelectric signals in real time. In other examples, processing circuitry 210 may periodically sample the one or more bioelectric signals according to a predetermined frequency or after a predetermined amount of time. In some examples, processing circuitry 210 may periodically sample the signal at a frequency of approximately 150 Hertz (Hz).

Processing circuitry 210 may include fixed function processing circuitry and/or programmable processing circuitry, and may comprise, for example, any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 210 herein may be embodied as firmware, hardware, software or any combination thereof. Processing circuitry 210 may control stimulation generation circuitry 202 according to electrical stimulation information 214 stored in memory 211 to apply particular stimulation parameter values specified by one or more of programs, such as voltage amplitude or current amplitude, pulse width, and/or pulse rate.

In the example shown in FIG. 2, the set of electrodes 116 includes electrodes 116A, 116B, 116C, and 116D, and the set of electrodes 118 includes electrodes 118A, 118B, 118C, and 118D. Stimulation generation circuitry 202 may be a single channel or multi-channel stimulation generator. In particular, stimulation generation circuitry 202 may be capable of delivering a single stimulation pulse, multiple stimulation pulses, or a continuous signal at a given time via a single electrode combination or multiple stimulation pulses at a given time via multiple electrode combinations. In some examples, however, stimulation generation circuitry 202 may be configured to deliver multiple channels on a time-interleaved basis. For example, switch circuitry may serve to time divide the output of stimulation generation circuitry 202 across different electrode combinations at different times to deliver multiple programs or channels of stimulation energy to patient 112.

Stimulation generation circuitry 202 may comprise multiple independently controllable voltage or current sources and sinks that are coupled to respective electrodes to drive the electrodes as cathodes or anodes. For example, to activate electrode 116A as a cathode, stimulation generation circuitry 202 may turn on a current source that is connected to electrode 116A and specify an amount of electric current amplitude to be delivered with each pulse. To activate electrode 116B as an anode, stimulation generation circuitry 202 may turn on a current sink connected to electrode 116B, which causes electrode 116B to sink the amount of current sourced by electrode 116A. Stimulation generation circuitry 202 may time-mux different electrode combinations by turning on different sources and sinks, connected to other selected electrodes, at different times. Stimulation generation circuitry 202 may form multi-electrode combinations of multiple cathodes and/or multiple anodes (or one cathode and multiple anodes or one anode and multiple cathodes) by selectively turning on particular sources and sinks, with the total amount of current sourced by the current sources of the cathodes being equal to the total amount of current sunk by the current sinks of the anode.

In some examples, each source or sink is actually a set of multiple, parallel sources and sinks formed by branches of a current mirror circuit. The parallel sources and sinks can be selectively activated to dial up or down the amount of current for a given electrode. For example, if there are 64 parallel source branches, activating 32 of the source branches provides 32x the current delivered in the reference branch of the current mirror circuit. This is 50% of the maximum possible current that could be delivered (if all 64 branches were activated) by the electrode. The reference current may be adjustable.

In some cases, the current sources may be driven to provide a constant voltage amplitude. In such examples, the medical practitioner may set the desired voltage amplitude.

As describes, in some examples, electrodes 116, 118 may also be sensing electrodes. For sensing electrodes, switch circuitry (not shown) may selectively couple electrodes 116, 118 to sensing circuitry 204. Sensing circuitry 204 may then sense the voltage or current across pairs of electrodes 116, 118, including in some examples the housing of the IMD 106.

In accordance with examples described in this disclosure, processing circuitry 210 may receive the sensed biomarker signals from sensing circuitry 204. For instance, processing circuitry 210 may receive sensed biomarker signals with patient 112 in different patient states (e.g., moving and not moving). Based on the instances of the sensed biomarker signals, processing circuitry 210 may determine the patient state, and the therapy parameters of the electrical stimulation, where the therapy parameters are stored as electrical stimulation information 214.

Electrodes 116, 118 on respective leads 114 may be constructed of a variety of different designs. For example, one or both of leads 114 may include two or more electrodes at each longitudinal location along the length of the lead, such as multiple electrodes, e.g., arranged as segments, at different perimeter locations around the perimeter of the lead at each of the locations A, B, C, and D.

As an example, one or both of leads 114 may include radially-segmented DBS arrays (rDBSA) of electrodes. In the rDBSA, as one example, there may be a first ring electrode of electrodes 116 around the perimeter of lead 114A at a first longitudinal location on lead 114A (e.g., location A). Below the first ring electrode, there may be three segmented electrodes of electrodes 116 around the perimeter of lead 114A at a second longitudinal location on lead 114A (e.g., location B). Below the three segmented electrodes, there may be another set of three segmented electrodes of electrodes 116 around the perimeter of lead 114A at a third longitudinal location of lead 114A (e.g., location C). Below the three segmented electrodes, there may be a second ring electrode of electrodes 116 around the perimeter of lead 114A (e.g., location D). Electrodes 118 may be similarly positioned along lead 114B.

The above is one example of the rDBSA array of electrodes, and the example techniques should not be considered limited to such an example. There may be other configurations of electrodes for DBS. Moreover, the example techniques are not limited to DBS, and other electrode configurations are possible.

In one example, the electrodes 116, 118 may be electrically coupled to switch circuitry via respective wires that are straight or coiled within the housing of the lead and run to a connector at the proximal end of the lead. In another example, each of the electrodes 116, 118 of the leads 114 may be electrodes deposited on a thin film. The thin film may include an electrically conductive trace for each electrode that runs the length of the thin film to a proximal end connector. The thin film may then be wrapped (e.g., a helical wrap) around an internal member to form the leads 114. These and other constructions may be used to create a lead with a complex electrode geometry.

Telemetry circuitry 208 supports wireless communication between IMD 106 and an external programmer 104 or another computing device under the control of processing circuitry 210. Processing circuitry 210 of IMD 106 may receive, as updates to programs, values for various stimulation parameters such as magnitude and electrode combination, from programmer 104 via telemetry circuitry 208. The updates to the therapy programs may be stored within electrical stimulation information 214 portion of memory 211. Telemetry circuitry 208 in IMD 106, as well as telemetry modules in other devices and systems described herein, such as programmer 104, may accomplish communication by radiofrequency (RF) communication techniques. In addition, telemetry circuitry 208 may communicate with external medical device programmer 104 via proximal inductive interaction of IMD 106 with programmer 104. Accordingly, telemetry circuitry 208 may send information to external programmer 104 on a continuous basis, at periodic intervals, or upon request from IMD 106 or programmer 104.

Power source 220 delivers operating power to various components of IMD 106. Power source 220 may include a small rechargeable or non-rechargeable battery and a power generation circuit to produce the operating power. Recharging may be accomplished through proximal inductive interaction between an external charger and an inductive charging coil within IMD 106. In some examples, power requirements may be small enough to allow IMD 106 to utilize patient motion and implement a kinetic energy-scavenging device to trickle charge a rechargeable battery. In other examples, traditional batteries may be used for a limited period of time.

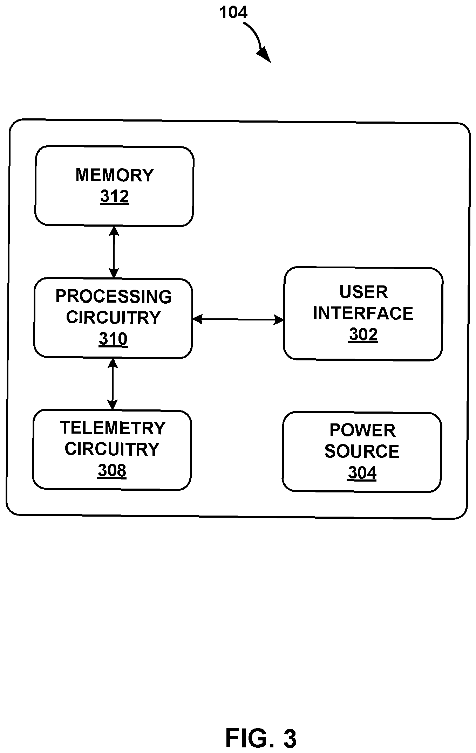

FIG. 3 is a block diagram of the external programmer 104 of FIG. 1. Although programmer 104 may generally be described as a hand-held device, programmer 104 may be a larger portable device or a more stationary device. In addition, in other examples, programmer 104 may be included as part of an external charging device or include the functionality of an external charging device. As illustrated in FIG. 3, programmer 104 may include processing circuitry 310, memory 312, user interface 302, telemetry circuitry 308, and power source 304. Memory 312 may store instructions that, when executed by processing circuitry 310, cause processing circuitry 310 and external programmer 104 to provide the functionality ascribed to external programmer 104 throughout this disclosure. Each of these components, or modules, may include electrical circuitry that is configured to perform some or all of the functionality described herein. For example, processing circuitry 310 may include processing circuitry configured to perform the processes discussed with respect to processing circuitry 310.

In general, programmer 104 comprises any suitable arrangement of hardware, alone or in combination with software and/or firmware, to perform the techniques attributed to programmer 104, and processing circuitry 310, user interface 302, and telemetry circuitry 308 of programmer 104. In various examples, programmer 104 may include one or more processors, which may include fixed function processing circuitry and/or programmable processing circuitry, as formed by, for example, one or more microprocessors, DSPs, ASICs, FPGAs, or any other equivalent integrated or discrete logic circuitry, as well as any combinations of such components. Programmer 104 also, in various examples, may include a memory 312, such as RAM, ROM, PROM, EPROM, EEPROM, flash memory, a hard disk, a CD-ROM, comprising executable instructions for causing the one or more processors to perform the actions attributed to them. Moreover, although processing circuitry 310 and telemetry circuitry 308 are described as separate modules, in some examples, processing circuitry 310 and telemetry circuitry 308 may be functionally integrated with one another. In some examples, processing circuitry 310 and telemetry circuitry 308 correspond to individual hardware units, such as ASICs, DSPs, FPGAs, or other hardware units.

Memory 312 (e.g., a storage device) may store instructions that, when executed by processing circuitry 310, cause processing circuitry 310 and programmer 104 to provide the functionality ascribed to programmer 104 throughout this disclosure. For example, memory 312 may include instructions that cause processing circuitry 310 to obtain a parameter set from memory or receive a user input and send a corresponding command to IMD 106, or instructions for any other functionality. In addition, memory 312 may include a plurality of programs, where each program includes a parameter set that defines stimulation therapy.

User interface 302 may include a button or keypad, lights, a speaker for voice commands, a display, such as a liquid crystal (LCD), light-emitting diode (LED), or organic light-emitting diode (OLED). In some examples the display may be a touch screen. User interface 302 may be configured to display any information related to the delivery of stimulation therapy, identified patient behaviors, sensed patient parameter values, patient behavior criteria, or any other such information. User interface 302 may also receive user input. The input may be, for example, in the form of pressing a button on a keypad or selecting an icon from a touch screen.

Telemetry circuitry 308 may support wireless communication between IMD 106 and programmer 104 under the control of processing circuitry 310. Telemetry circuitry 308 may also be configured to communicate with another computing device via wireless communication techniques, or direct communication through a wired connection. In some examples, telemetry circuitry 308 provides wireless communication via an RF or proximal inductive medium. In some examples, telemetry circuitry 308 includes an antenna, which may take on a variety of forms, such as an internal or external antenna.

Examples of local wireless communication techniques that may be employed to facilitate communication between programmer 104 and IMD 106 include RF communication according to the 802.11 or Bluetooth specification sets or other standard or proprietary telemetry protocols. In this manner, other external devices may be capable of communicating with programmer 104 without needing to establish a secure wireless connection.

In some examples, processing circuitry 310 of external programmer 104 defines the parameters of electrical stimulation therapy, stored in memory 312, for delivering adaptive DBS to patient 112. In one example, processing circuitry 310 of external programmer 104, via telemetry circuitry 308, issues commands to IMD 106 causing IMD 106 to deliver electrical stimulation therapy via electrodes 116, 118 via leads 114. In some examples, processing circuitry 310 may be configured to perform various operations described with respect to processing circuitry 210. For example, processing circuitry 210, via telemetry circuitry 208, may output sensed biomarker signals to programmer 104. Processing circuitry 310 may determine whether IMD 106 should deliver therapy in accordance with a first closed-loop therapy mode (e.g., normal closed-loop therapy mode) or a second closed-loop therapy mode (e.g., the polling closed-loop therapy mode). In this disclosure, the example techniques described with reference to processing circuitry refer to example techniques that may be performed by processing circuitry 210, processing circuitry 310, some other processing circuitry (e.g., in the cloud, on a server, on a computer, etc.), or any combination of such processing circuitry.

In some examples described above, a medical practitioner in a clinic setting may determine whether patient 112 should receive therapy in accordance with the first closed-loop therapy mode or the second closed-loop therapy mode. However, the example techniques are not so limited. In some examples, processing circuitry 310 or processing circuitry 210 may determine whether patient 112 should receive therapy in accordance with the first closed-loop therapy mode or the second closed-loop therapy mode.

In some cases, overtime the efficacy of the therapy may change (e.g., such as changes to the body of patient 112). Accordingly, periodic adjustment to therapy and evaluation of effectiveness of closed-loop therapy may be appropriate. Patient 112 may benefit from such adjustments occurring dynamically without needing to visit the clinician. In some examples, changes to the therapy, especially changes that result in therapy outside a range, may require clinician confirmation before the change is made.

As an example, IMD 106 may be configured to deliver therapy in accordance with the first closed-loop therapy mode (e.g., normal closed-loop therapy mode). At some point after, processing circuitry 210 or processing circuitry 310 may determine that the sensed biomarker signals appear to be in a noise level, may determine that therapy parameters are not being changed, or may periodically recheck if the therapy mode should be changed. In response, processing circuitry 310 may cause user interface 302 to output a message requesting patient 112 to be in a first patient state. Patient 112 may provide an input indicating that patient 112 is in the first patient state, or confirmation that patient 112 is in the first patient state may be received from other sensors of IMD 106. Processing circuitry 210 may receive a first instance of the biomarker signal with stimulation on.

Processing circuitry 310 may cause user interface 302 to output a message requesting patient 112 to be in a second patient state. Patient 112 may provide an input indicating that patient 112 is in the second patient state, or confirmation that patient 112 is in the second patient state may be received from other sensors of IMD 106. Processing circuitry 210 may receive a second instance of the biomarker signal with stimulation on.

Processing circuitry 210 and/or processing circuitry 310 may determine if there is a difference in the first instance and the second instance of the biomarker signal. If the difference is statistically significant, processing circuitry 210 and/or processing circuitry 310 may select the first closed-loop therapy mode (e.g., normal closed-loop therapy mode). If the difference is not statistically significant, processing circuitry 210 and/or processing circuitry 310 may select the second closed-loop therapy mode (e.g., polling closed-loop therapy mode). In some examples, before selecting the second closed-loop therapy mode, processing circuitry 210 and/or processing circuitry 310 may adjust therapy parameters to determine if it is possible to provide therapy in accordance with the first closed-loop therapy mode.

The above describes an example where patient 112 started in the first closed-loop therapy mode, and a determination was made whether to transition patient 112 to the second closed-loop therapy mode. In some examples, patient 112 may start in the second closed-loop therapy mode (e.g., polling closed-loop therapy mode), and a determination may be made about transitioning patient 112 to the first closed-loop therapy mode (e.g., normal closed-loop therapy mode).

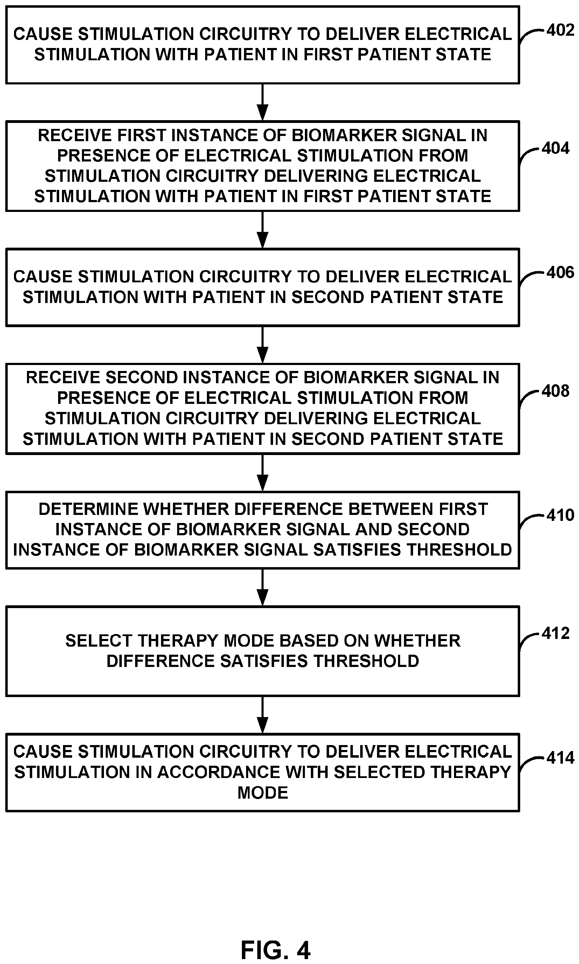

FIG. 4 is a flowchart illustrating an example operation for selecting a therapy mode, in accordance with one or more examples described in this disclosure. For ease of illustration, the example techniques of FIGS. 4-7 are described with respect to processing circuitry 210. However, the example techniques may be performed by processing circuitry 210, processing circuitry 310, some other processing circuitry, or a combination of any such processing circuitry.

Processing circuitry 210 may cause stimulation generation circuitry 202 to deliver electrical stimulation with patient 112 in a first patient state (402) (e.g., patient 112 is detected as being or known to be in the first patient state). For instance, shortly after surgery or possibly even after patient 112 has been receiving therapy for a while and would like to update therapy, a medical practitioner or an output from programmer 104 may request patient 112 to be in a first patient state (e.g., not moving), and when patient 112 is in the first patient state, stimulation generation circuitry 202 may deliver electrical stimulation to patient 112.

Processing circuitry 210 may receive a first instance of a biomarker signal of sensed one or more biomarker signals in presence of electrical stimulation from (e.g., after or in response to) stimulation generation circuitry 202 delivering the electrical stimulation with patient 112 in the first patient state (404). For instance, sensing circuitry 204 may be configured to sense one or more biomarker signals, and processing circuitry 210 may receive the first instance of the biomarker signal from sensing circuitry 204. Examples of the biomarker signal include a local field potential (LFP) signal, an evoked compound action potential (ECAP) signal, and an electromyography (EMG) signal. In general, the biomarker signals may be physiological signals generated from nerves or muscles within the body of patient 112 (e.g., intrinsic signals). Processing circuitry 210 may store information indicative of the first instance of the biomarker signal in memory 211.

Processing circuitry 210 may cause stimulation generation circuitry 202 to deliver electrical stimulation with patient 112 in a second patient state (406) (e.g., patient 112 is detected as being or known to be in the second patient state). For instance, after stimulation generation circuitry 202 delivers electrical stimulation with patient 112 in the first patient state and sensing circuitry 204 sensed the first instance of the biomarker signal, a medical practitioner or programmer 104 may request patient 112 to be in a second patient state (e.g., moving). When patient 112 is in the second patient state, stimulation generation circuitry 202 may deliver electrical stimulation to patient 112.

Processing circuitry 210 may receive a second instance of a biomarker signal of sensed one or more biomarker signals in presence of electrical stimulation from (e.g., after or in response to) stimulation generation circuitry 202 delivering the electrical stimulation with patient 112 in the second patient state (408). The first instance of the biomarker signal and the second instance of the biomarker signal may be for the same biomarker signal, but different instances. In some examples, it may be possible for first instance of the biomarker signal and the second instance of the biomarker signal to be for different biomarker signals. Processing circuitry 210 may store information indicative of the second instance of the biomarker signal in memory 211.

Processing circuitry 210 may determine whether a difference between the first instance of the biomarker signal and the second instance of the biomarker signal satisfies a threshold (410). As one example, processing circuitry 210 may determine whether the difference is less than a threshold to determine whether the difference between the first instance of the biomarker signal and the second instance of the biomarker signal satisfies the threshold. For example, processing circuitry 210 may determine a ratio between the power of the beta band (e.g., 13-38 Hz) to the power of the gamma band (e.g., 39-42 Hz or 50-90 Hz). The ratio may be indicative of sleep or awake, on or off medication, or moving or not moving.

In this example, processing circuitry 210 may determine a first instance of the ratio between the power of the beta band to the power of the gamma band (e.g., first instance of the biomarker signal), and a second instance of the ratio between the power of the beta band to the power of the gamma band (e.g., second instance of the biomarker signal). Processing circuitry 210 may determine a difference between the first instance of the ratio and the second instance of the ratio. In general, processing circuitry 210 may determine whether the difference between the first instance of the biomarker signal and the second instance of the biomarker signal is statistically significant as a way to determine whether a difference between the first instance of the biomarker signal and the second instance of the biomarker signal satisfies the threshold.

Processing circuitry 210 may be configured to select a therapy mode for electrical stimulation delivery based on whether the difference between the first instance of the biomarker signal and the second instance of the biomarker signal satisfies the threshold (412). One example of the therapy mode is the first closed-loop therapy mode (e.g., normal closed-loop therapy mode). Another example of the therapy mode is the second closed-loop therapy mode (e.g., polling closed-loop therapy mode).

For example, in a first case, processing circuitry 210 may determine that the difference between the first instance of the biomarker signal and the second instance of the biomarker signal satisfies the threshold (e.g., the difference is statistically significant). In the first case, processing circuitry 210 may select the first closed-loop therapy mode. In the first case, because the difference between the first instance of the biomarker signal and the second instance of the biomarker signal is statistically significant, it may be possible for processing circuitry 210 to determine that patient 112 transitioned from the first patient state to the second patient state, and vice-versa based on the biomarker signal. Therefore, even with stimulation being delivered to patient 112, the electrical stimulation is not confounding or suppressing the biomarker signal to a level at which processing circuitry 210 cannot differentiate between the first instance of the biomarker signal and the second instance of the biomarker signal.

In a second case, processing circuitry 210 may determine that the difference between the first instance of the biomarker signal and the second instance of the biomarker signal does not satisfy the threshold (e.g., the difference is not statistically significant). In the second case, processing circuitry 210 may select the second closed-loop therapy mode. In the second case, because the difference between the first instance of the biomarker signal and the second instance of the biomarker signal is not statistically significant, it may not be possible for processing circuitry 210 to determine that patient 112 transitioned from the first patient state to the second patient state, and vice-versa based on the biomarker signal. That is, with stimulation being delivered to patient 112, the electrical stimulation is confounding or suppressing the biomarker signal to a level at which processing circuitry 210 cannot differentiate between the first instance of the biomarker signal and the second instance of the biomarker signal. Accordingly, for the second closed-loop therapy mode, processing circuitry 210 may temporally (e.g., less than 1 second, less than 500 milliseconds, less than 1 millisecond, less than 500 micro-seconds, or less than 100 micro-seconds) cease or at least change the electrical stimulation that is being delivered to patient 112 to allow the biomarker signal to not be confounded or suppressed, so that processing circuitry 210 can differentiate between the first instance of the biomarker signal and a second instance of the biomarker signal.

Processing circuitry 210 may cause stimulation generation circuity 202 to deliver electrical stimulation in accordance with the selected therapy mode (414). For instance, stimulation generation circuitry 202 may deliver electrical stimulation in accordance with the first closed-loop therapy mode or the second closed-loop therapy mode.

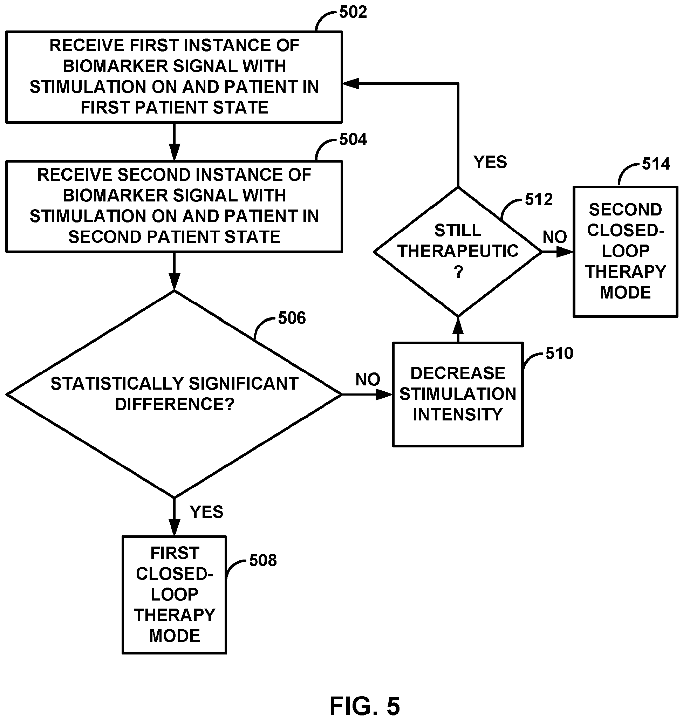

FIG. 5 is a flowchart illustrating another example operation for selecting a therapy mode, in accordance with one or more examples described in this disclosure. The example techniques of FIG. 5 may be similar to those of FIG. 4, but further illustrate examples where processing circuitry 210 may iterate the amount of electrical stimulation that is delivered to determine whether to select the first closed-loop therapy mode or second closed-loop therapy mode.

Processing circuitry 210 may receive a first instance of a biomarker signal with stimulation on and patient 112 in a first patient state (502), and receive a second instance of the biomarker signal with stimulation on and patient 112 in a second patient state (504). Processing circuitry 210 may determine whether the difference between the first instance of the biomarker signal and the second instance of the biomarker signal is statistically significant (506). In a first case where the difference between the first instance of the biomarker signal and the second instance of the biomarker signal is statistically significant (YES of 506), processing circuitry 210 may select the first closed-loop therapy mode (e.g., normal closed-loop therapy mode) (508).

However, in a second case where the difference between the first instance of the biomarker signal and the second instance of the biomarker signal is not statistically significant (NO of 506), indicating possible confounding or suppressing of the biomarker signal, processing circuitry 210 may adjust the therapy parameters to reduce the intensity of the electrical stimulation (510). As one example, processing circuitry 210 may reduce the amplitude of the electrical stimulation. As another example, processing circuitry 210 may reduce the pulse width of the electrical stimulation.

Processing circuitry 210 (or possibly processing circuitry 310) may determine whether the electrical stimulation having the reduced stimulation intensity is still therapeutic (512). For instance, patient 112 may provide feedback as to whether patient 112 is feeling therapeutic effect. In examples of movement disorders, the medical practitioner may visibly determine if there is a change in involuntary movements.

If patient 112 is experiencing therapeutic effect (YES of 512), processing circuitry 210 may repeat the process of determining if there is a statistically significant difference between the first instance of the biomarker signal and the second instance of biomarker signal. For instance, it may be possible that with the reduced stimulation intensity but still providing therapeutic effect, the stimulation signal does not substantially confound or suppress the biomarker signal. In this case, it may be possible to provide electrical stimulation in accordance with the first closed-loop therapy mode.

Processing circuitry 210 may repeat the operations of decreasing stimulation intensity, determining if therapy is still therapeutic, and determining if the first closed-loop therapy mode is possible until processing circuitry 210 decreases the stimulation intensity to a level where the stimulation is no longer therapeutic. For instance, if patient 112 is no longer experiencing therapeutic effect (NO of 512), in this second case, processing circuitry 210 may select the second closed-loop therapy mode (e.g., polling closed-loop therapy mode) (514).

Accordingly, in FIG. 5, prior to selecting the second closed-loop therapy mode, processing circuitry 210 is configured to reduce intensity of the electrical stimulation (510), and determine that electrical stimulation having reduced intensity is not therapeutic (NO of 512). In such cases, to select the second closed-loop therapy mode (514), processing circuitry 210 is configured to select the second closed-loop therapy mode based on the determination that the electrical stimulation having reduced intensity is not therapeutic (e.g., based on NO of 512).

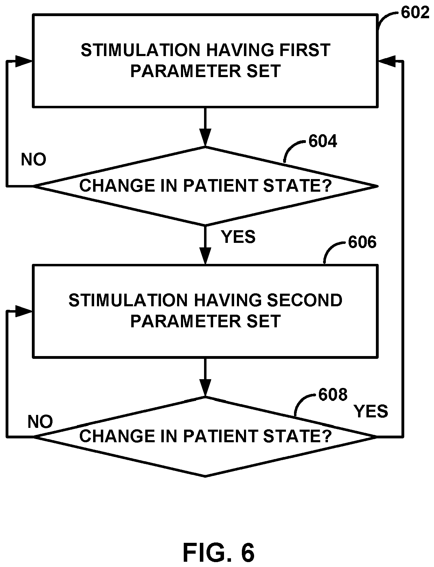

FIG. 6 is a flowchart illustrating an example operation of therapy delivery in accordance with a first closed-loop therapy mode. An example of the first closed-loop therapy mode is a normal closed-loop therapy mode. FIG. 6 illustrates a first case in which the normal closed-loop therapy mode is selected.

In some examples, to perform the example techniques of FIG. 6, processing circuitry 210 may determine that the difference between the first instance of the biomarker signal and the second instance of the biomarker signal satisfies the threshold. Also, to select the therapy mode for electrical stimulation delivery, processing circuitry 210 may be configured to select a normal closed-loop therapy mode. In the example of FIG. 6, to cause the stimulation generation circuitry 202 to deliver electrical stimulation in accordance with the selected therapy mode, processing circuitry 210 may be configured to cause stimulation generation circuitry 202 to transition between delivering electrical stimulation having a first parameter set based on patient 112 being in the first patient state and delivering electrical stimulation having a second parameter set based on patient 112 being in the second patient state.

For example, processing circuitry 210 may cause stimulation circuitry 202 to deliver electrical stimulation having a first parameter set (602). Processing circuitry 210 may determine whether there is a change in patient state (604). For instance, processing circuitry 210 may determine if there is a change in a biomarker signal indicating a change in patient state.

If there is no change in the patient state (NO of 604), processing circuitry 210 may cause stimulation generation circuitry 202 to keep delivering electrical stimulation having the first parameter set (602). If there is change in the patient state (YES of 604), processing circuitry 210 may transition from electrical stimulation having the first parameter set to causing stimulation generation circuitry 202 to deliver electrical stimulation having a second parameter set (606).

Processing circuitry 210 may determine whether there is a change in patient state (608). For instance, processing circuitry 210 may determine if there is a change in a biomarker signal indicating a change in patient state.

If there is no change in the patient state (NO of 608), processing circuitry 210 may cause stimulation generation circuitry 202 to keep delivering electrical stimulation having the second parameter set (606). If there is change in the patient state (YES of 608), processing circuitry 210 may transition from electrical stimulation having the second parameter set to causing stimulation generation circuitry 202 to deliver electrical stimulation having the first parameter set (602).

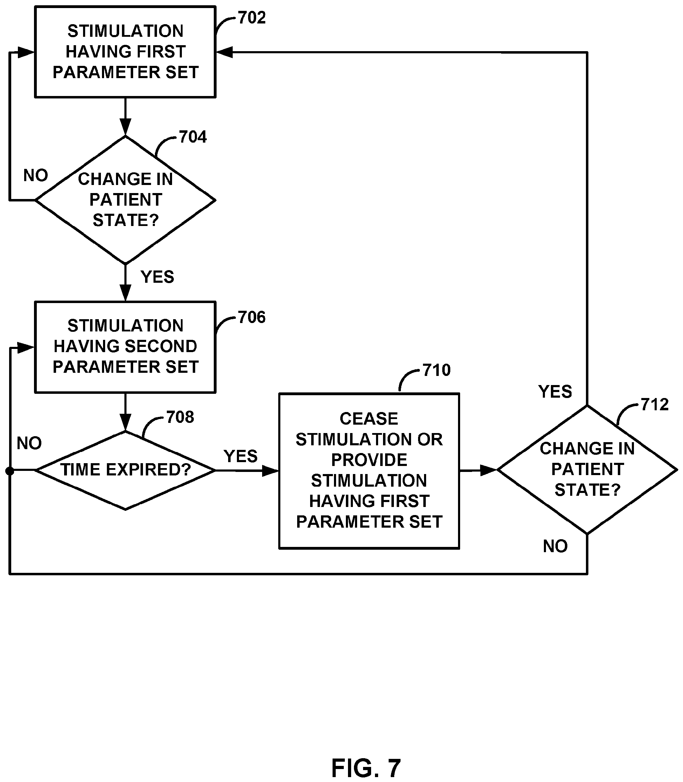

FIG. 7 is a flowchart illustrating an example operation of therapy delivery in accordance with a second closed-loop therapy mode. An example of the second closed-loop therapy mode is the polling closed-loop therapy mode. FIG. 7 illustrates a second case in which the polling closed-loop therapy mode is selected.

In some examples, to perform the example techniques of FIG. 7, processing circuitry 210 may determine that the difference between the first instance of the biomarker signal and the second instance of the biomarker signal does not satisfy the threshold. Also, to select the therapy mode for electrical stimulation delivery, processing circuitry 210 may be configured to select a polling closed-loop therapy mode.

In FIG. 7, processing circuitry 210 may cause stimulation generation circuitry 202 to deliver electrical stimulation having a first parameter set based on the patient being in the first patient state (702). Processing circuitry 210 may determine whether patient 112 transitioned to the second patient state (704). If processing circuitry 210 determine that patient 112 did not transition to the second patient state (NO of 704), processing circuitry 210 may cause stimulation generation circuitry 202 to keep delivering electrical stimulation having the first parameter set (702).

If processing circuitry 210 determined that patient 112 did transition to the second patient state (YES of 704), processing circuitry 210 may cause stimulation generation circuitry 202 to deliver electrical stimulation having a second parameter set based on the patient being in the second patient state (706). In the example of FIG. 7, delivering electrical stimulation having the first parameter set may not substantially confound or suppress the biomarker signal. Therefore, processing circuitry 210 may be able to determine when patient 112 transitioned from the first patient state to the second patient state based on the biomarker signal. However, in the example of FIG. 7, delivery of electrical stimulation having the second parameter set may confound or suppress the biomarker signal. Therefore, processing circuitry 210 may not be able to determine when patient 112 transitioned from the second patient state to the first patient state based on the biomarker signal with electrical stimulation having the second parameter set being delivered to patient 112.

In the example of FIG. 7, processing circuitry 210 may determine if it is time to check if patient 112 has transitioned from the second patient state back to the first patient state. For instance, processing circuitry 210 may periodically check if patient 112 has transitioned from the second patient state to the first patient state. To perform such checking, processing circuitry 210 may maintain a timer, and may determine if the timer has expired (708). The timer may be set to check if patient 112 has transitioned from the second patient state to the first patient state every minute, every 5 minutes, every 10 minutes, every hour, or more and anytime in between.

If the timer has not expired (NO of 708), processing circuitry 210 may keep causing stimulation generation circuitry 202 to deliver electrical stimulation having the second parameter set (706). After the stimulation generation circuitry delivers electrical stimulation having the second parameter set for a period of time (e.g., YES of 708), processing circuitry 210 may temporally cause stimulation generation circuitry 202 to cease delivery of electrical stimulation or to deliver electrical stimulation having the first parameter set (710).

During cessation of electrical stimulation or stimulation generation circuitry 202 delivering electrical stimulation having the first parameter set, processing circuitry 210 may determine whether patient 112 transitioned to the first patient state, i.e., whether there is a change in patient state (712). Processing circuitry 210 may cause stimulation generation circuitry 202 to deliver electrical stimulation having the first parameter set or deliver electrical stimulation having the second parameter set based on the determination of whether the patient transitioned to the first patient state. For example, if patient 112 transitioned from the second patient state to the first patient state (YES of 712), processing circuitry 210 may deliver electrical stimulation having the first parameter set (702). If patient 112 did not transition from the second patient state to the first patient state (NO of 712), processing circuitry 210 may deliver electrical stimulation having the second parameter set (706).

FIGS. 6 and 7 illustrate examples with first parameter set and second parameter set based on two patient states of whether the patient is moving. However, the example techniques are not so limited. As another example, the first patient state may be the patient having taken medication, and the second patient state may be one of the steady state of the medication (e.g., when the medication is effective) or when the medication is no longer effective.

In some examples, there may be multiple parameter sets and multiple patient states. For instance, patient states may be based on medication. A first patient state is when patient just took medication (i.e., onset). A second patient state is steady state of effectiveness of medication. A third patient state may be when medication is no longer effective (e.g., as if no medication is present). In such an example, there may be three potential parameter sets that are desired to be delivered for the three patient states. That is, processing circuitry 210 may cycle through the different parameter sets based on whether the patient is in the first, second, or third patient state.

There may be more than three patient states and three parameter sets. In some examples, the patient states may be based on a combination of medication and movement. For instance, there may be a matrix of states based on different patient conditions (e.g., medication, movement, and so forth). There may be parameter sets for each of the states in the matrix of states. Accordingly, the above example of two parameter sets and two patient states should not be considered limiting.

The techniques described in this disclosure may be implemented, at least in part, in hardware, software, firmware, or any combination thereof. For example, various aspects of the described techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a computer-readable medium, such as a computer-readable storage medium, containing instructions. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

Various examples have been described. The invention is defined by the following claims.

## Claims

1. A system for electrical stimulation delivery, the system comprising:
stimulation generation circuitry (202) configured to deliver electrical stimulation to a patient;
sensing circuitry (204) configured to sense one or more biomarker signals; and
processing circuitry (210) configured to:
cause the stimulation generation circuitry (202) to deliver electrical stimulation with the patient in a first patient state;
receive a first instance of a biomarker signal of the sensed one or more biomarker signals in presence of the electrical stimulation from the stimulation generation circuitry (202) delivering the electrical stimulation with the patient in the first patient state;
cause the stimulation generation circuitry (202) to deliver electrical stimulation with the patient in a second patient state;
receive a second instance of the biomarker signal of the sensed one or more biomarker signals in presence of the electrical stimulation from the stimulation generation circuitry (202) delivering the electrical stimulation with the patient in the second patient state;
determine whether a difference between the first instance of the biomarker signal and the second instance of the biomarker signal satisfies a threshold;
select a therapy mode for electrical stimulation delivery based on whether the difference between the first instance of the biomarker signal and the second instance of the biomarker signal satisfies the threshold; and
cause the stimulation generation circuitry (202) to deliver electrical stimulation in accordance with the selected therapy mode.

2. The system of claim 1, wherein at least one of:
(1) the first patient state is the patient not moving, and the second patient state is the patient moving, and
(2) the first patient state is the patient having taken medication, and the second patient state is one of the steady state of the medication or when medication is no longer effective.

3. The system of any of the claims 1 or 2,
wherein to determine whether the difference between the first instance of the biomarker signal and the second instance of the biomarker signal satisfies the threshold, the processing circuitry (210) is configured to determine that the difference between the first instance of the biomarker signal and the second instance of the biomarker signal satisfies the threshold,
wherein to select the therapy mode for electrical stimulation delivery, the processing circuitry (210) is configured to select a normal closed-loop therapy mode, and
wherein to cause the stimulation generation circuitry (202) to deliver electrical stimulation in accordance with the selected therapy mode, the processing circuitry (210) is configured to:
cause the stimulation generation circuitry (202) to transition between delivering electrical stimulation having a first parameter set based on the patient being in the first patient state and delivering electrical stimulation having a second parameter set based on the patient being in the second patient state.

4. The system of any of the claims 1 or 2, wherein to determine whether the difference between the first instance of the biomarker signal and the second instance of the biomarker signal satisfies the threshold, the processing circuitry (210) is configured to determine that the difference between the first instance of the biomarker signal and the second instance of the biomarker signal does not satisfy the threshold,
wherein to select the therapy mode for electrical stimulation delivery, the processing circuitry (210) is configured to select a polling closed-loop therapy mode, and
wherein to cause the stimulation generation circuitry (202) to deliver electrical stimulation in accordance with the selected therapy mode, the processing circuitry (210) is configured to:
cause the stimulation generation circuitry (202) to deliver electrical stimulation having a first parameter set based on the patient being in the first patient state;
determine that the patient transitioned to the second patient state;
cause the stimulation generation circuitry (202) to deliver electrical stimulation having a second parameter set based on the patient being in the second patient state;
after the stimulation generation circuitry (202) delivers electrical stimulation having the second parameter set, temporally cause the stimulation generation circuitry (202) to cease delivery of electrical stimulation or to deliver electrical stimulation having the first parameter set;
during the cessation of electrical stimulation or the stimulation generation circuitry (202) delivering electrical stimulation having the first parameter set, determine whether the patient transitioned to the first patient state; and
cause the stimulation generation circuitry (202) to deliver electrical stimulation having the first parameter set or deliver electrical stimulation having the second parameter set based on the determination of whether the patient transitioned to the first patient state.

5. The system of claim 4,
wherein prior to selecting the polling closed-loop therapy mode, the processing circuitry (210) is configured to:
reduce intensity of the electrical stimulation; and
determine that electrical stimulation having the reduced intensity is not therapeutic, and
wherein to select the polling closed-loop therapy mode, the processing circuitry (210) is configured to select the polling closed-loop therapy mode based on the determination that the electrical stimulation having reduced intensity is not therapeutic.

6. The system of any of the preceding claims, wherein the first instance of the biomarker signal and the second instance of the biomarker signal comprise one of a local field potential (LFP) signal, an evoked compound action potential (ECAP) signal, and an electromyography (EMG) signal.

7. The system of any of the preceding claims, the system further comprising an implantable medical device (IMD), wherein the IMD includes the stimulation generation circuitry (202), the sensing circuitry (204), and the processing circuitry (210).

8. The system of any of the claims 1 to 6, the system further comprising an implantable medical device (IMD) and an external programmer, wherein the IMD includes the stimulation generation circuitry (202) and the sensing circuitry, and wherein the external programmer or a combination of the external programmer and the IMD include the processing circuitry (210).

9. A computer-readable storage medium storing instruction thereon that when executed cause one or more processors to perform a method for electrical stimulation delivery, the method comprising:
causing, with processing circuitry, a stimulation generation circuitry to deliver electrical stimulation with a patient in a first patient state;
receiving, with the processing circuitry, a first instance of a biomarker signal sensed by a sensing circuitry in presence of the electrical stimulation from the stimulation generation circuitry delivering the electrical stimulation with the patient in the first patient state;
causing, with the processing circuitry, the stimulation generation circuitry to deliver electrical stimulation with the patient in a second patient state;
receiving, with the processing circuitry, a second instance of the biomarker signal sensed by the sensing circuitry in presence of the electrical stimulation from the stimulation generation circuitry delivering the electrical stimulation with the patient in the second patient state;
determining, with the processing circuitry, whether a difference between the first instance of the biomarker signal and the second instance of the biomarker signal satisfies a threshold;
selecting, with the processing circuitry, a therapy mode for electrical stimulation delivery based on whether the difference between the first instance of the biomarker signal and the second instance of the biomarker signal satisfies the threshold; and
causing, with the processing circuitry, the stimulation generation circuitry to deliver electrical stimulation in accordance with the selected therapy mode.

10. The computer-readable storage medium of claim 9, wherein at least one of:
(1) the first patient state is the patient not moving, and the second patient state is the patient moving, and
(2) the first patient state is the patient having taken medication, and the second patient state is one of the steady state of the medication or when medication is no longer effective.

11. The computer-readable storage medium of any of the claims 9 or 10,
wherein determining whether the difference between the first instance of the biomarker signal and the second instance of the biomarker signal satisfies the threshold comprises determining that the difference between the first instance of the biomarker signal and the second instance of the biomarker signal satisfies the threshold,
wherein selecting the therapy mode for electrical stimulation delivery comprises selecting a normal closed-loop therapy mode, and
wherein causing the stimulation generation circuitry to deliver electrical stimulation in accordance with the selected therapy mode comprises:
causing the stimulation generation circuitry to transition between delivering electrical stimulation having a first parameter set based on the patient being in the first patient state and delivering electrical stimulation having a second parameter set based on the patient being in the second patient state.

12. The computer-readable storage medium of any of the claim 9 or 10, wherein determining whether the difference between the first instance of the biomarker signal and the second instance of the biomarker signal satisfies the threshold comprises determining that the difference between the first instance of the biomarker signal and the second instance of the biomarker signal does not satisfy the threshold,
wherein selecting the therapy mode for electrical stimulation delivery comprises selecting a polling closed-loop therapy mode, and
wherein causing the stimulation generation circuitry to deliver electrical stimulation in accordance with the selected therapy mode comprises:
causing the stimulation generation circuitry to deliver electrical stimulation having a first parameter set based on the patient being in the first patient state;
determining that the patient transitioned to the second patient state;
causing the stimulation generation circuitry to deliver electrical stimulation having a second parameter set based on the patient being in the second patient state;
after the stimulation generation circuitry delivers electrical stimulation having the second parameter set, temporally causing the stimulation generation circuitry to cease delivery of electrical stimulation or to deliver electrical stimulation having the first parameter set;
during the cessation of electrical stimulation or stimulation generation circuitry delivering electrical stimulation having the first parameter set, determining whether the patient transitioned to the first patient state; and
causing the stimulation generation circuitry to deliver electrical stimulation having the first parameter set or deliver electrical stimulation having the second parameter set based on the determination of whether the patient transitioned to the first patient state.

13. The computer-readable storage medium of claim 12, further comprising:
prior to selecting the polling closed-loop therapy mode:
reducing intensity of the electrical stimulation; and
determining that electrical stimulation having reduced intensity is not therapeutic, and
wherein selecting the polling closed-loop therapy mode comprises selecting the polling closed-loop therapy mode based on the determination that the electrical stimulation having reduced intensity is not therapeutic.

14. The computer-readable storage medium of any of the claims 9 to 13, wherein the first instance of the biomarker signal and the second instance of the biomarker signal comprise one of a local field potential (LFP) signal, an evoked compound action potential (ECAP) signal, and an electromyography (EMG) signal.

15. The computer-readable storage medium of any of the claim 9 to 14, wherein an implantable medical device (IMD) includes the stimulation generation circuitry, the sensing circuitry, and the processing circuitry.

## Patentansprüche

1. System für eine Abgabe einer elektrischen Stimulation, das System umfassend:
Stimulationserzeugungsschaltlogik (202), die konfiguriert ist, um die elektrische Stimulation an einen Patienten abzugeben;
Erfassungsschaltlogik (204), die konfiguriert ist, um ein oder mehrere Biomarker-Signale zu erfassen; und
Verarbeitungsschaltlogik (210), die konfiguriert ist zum:
Veranlassen der Stimulationserzeugungsschaltlogik (202), die elektrische Stimulation mit dem Patienten in einem ersten Patientenzustand abzugeben;
Empfangen einer ersten Instanz eines Biomarker-Signals des erfassten einen oder der mehreren Biomarker-Signale in Anwesenheit der elektrischen Stimulation von der Stimulationserzeugungsschaltlogik (202), die die elektrische Stimulation mit dem Patienten in dem ersten Patientenzustand abgibt;
Veranlassen der Stimulationserzeugungsschaltlogik (202), die elektrische Stimulation mit dem Patienten in einem zweiten Patientenzustand abzugeben;
Empfangen einer zweiten Instanz des Biomarker-Signals des erfassten einen oder der mehreren Biomarker-Signale in Anwesenheit der elektrischen Stimulation von der Stimulationserzeugungsschaltlogik (202), die die elektrische Stimulation mit dem Patienten in dem zweiten Patientenzustand abgibt;
Bestimmen, ob eine Differenz zwischen der ersten Instanz des Biomarker-Signals und der zweiten Instanz des Biomarker-Signals eine Schwelle erfüllt;
Auswählen eines Therapiemodus für die Abgabe der elektrischen Stimulation, basierend darauf, ob die Differenz zwischen der ersten Instanz des Biomarker-Signals und der zweiten Instanz des Biomarker-Signals die Schwelle erfüllt; und
Veranlassen der Stimulationserzeugungsschaltlogik (202), die elektrische Stimulation gemäß dem ausgewählten Therapiemodus abzugeben.

2. Verfahren nach Anspruch 1, wobei mindestens eines von:
(1) der erste Patientenzustand ist, dass der Patient sich nicht bewegt, und der zweite Patientenzustand ist, dass der Patient sich bewegt, und
(2) der erste Patientenzustand ist, dass der Patient ein Arzneimittel eingenommen hat und der zweite Patientenzustand einer des stabilen Zustands des Arzneimittels ist oder wenn das Arzneimittel nicht mehr wirkt.

3. System nach einem der Ansprüche 1 oder 2,
wobei, um zu bestimmen, ob die Differenz zwischen der ersten Instanz des Biomarker-Signals und der zweiten Instanz des Biomarker-Signals die Schwelle erfüllt, die Verarbeitungsschaltlogik (210) konfiguriert ist, um zu bestimmen, dass die Differenz zwischen der ersten Instanz des Biomarker-Signals und der zweiten Instanz des Biomarker-Signals die Schwelle erfüllt,
wobei, um den Therapiemodus für die Abgabe der elektrischen Stimulation auszuwählen, die Verarbeitungsschaltlogik (210) konfiguriert ist, um einen Therapiemodus mit einem normal geschlossenen Regelkreis auszuwählen, und
wobei, um die Stimulationserzeugungsschaltlogik (202) zu veranlassen, die elektrische Stimulation gemäß dem ausgewählten Therapiemodus abzugeben, die Verarbeitungsschaltlogik (210) konfiguriert ist, zum:
Veranlassen der Stimulationserzeugungsschaltlogik (202), zwischen dem Abgeben der elektrischen Stimulation, die einen ersten Parametersatz aufweist, basierend darauf, dass der Patient sich in dem ersten Patientenzustand befindet, und dem Abgeben der elektrischen Stimulation, die einen zweiten Parametersatz aufweist, basierend darauf, dass der Patienten sich in dem zweiten Patientenzustand befindet, überzugehen.

4. System nach einem der Ansprüche 1 oder 2, wobei, um zu bestimmen, ob die Differenz zwischen der ersten Instanz des Biomarker-Signals und der zweiten Instanz des Biomarker-Signals die Schwelle erfüllt, die Verarbeitungsschaltlogik (210) konfiguriert ist, um zu bestimmen, dass die Differenz zwischen der ersten Instanz des Biomarker-Signals und der zweiten Instanz des Biomarker-Signals die Schwelle nicht erfüllt,
wobei, um den Therapiemodus für die Abgabe der elektrischen Stimulation auszuwählen, die Verarbeitungsschaltlogik (210) konfiguriert ist, um einen Abfragetherapiemodus mit dem geschlossenen Regelkreis auszuwählen, und
wobei, um die Stimulationserzeugungsschaltlogik (202) zu veranlassen, die elektrische Stimulation gemäß dem ausgewählten Therapiemodus abzugeben, die Verarbeitungsschaltlogik (210) konfiguriert ist, zum:
Veranlassen der Stimulationserzeugungsschaltlogik (202), die elektrische Stimulation, die einen ersten Parametersatz aufweist, basierend darauf, dass der Patient sich in dem ersten Patientenzustand befindet, abzugeben;
Bestimmen, dass der Patient in den zweiten Patientenzustand übergegangen ist;
Veranlassen der Stimulationserzeugungsschaltlogik (202), die elektrische Stimulation, die einen zweiten Parametersatz aufweist, basierend darauf, dass der Patient sich in dem zweiten Patientenzustand befindet, abzugeben;
vorübergehenden Veranlassen der Stimulationserzeugungsschaltlogik (202), die Abgabe der elektrischen Stimulation einzustellen oder die elektrische Stimulation, die den ersten Parametersatz aufweist, abzugeben, nachdem die Stimulationserzeugungsschaltlogik (202) die elektrische Stimulation, die den zweiten Parametersatz aufweist, abgibt;
Bestimmen, während der Einstellung der elektrischen Stimulation oder der Stimulationserzeugungsschaltlogik (202), die die elektrische Stimulation, die den ersten Parametersatz aufweist, abgibt, ob der Patient in den ersten Patientenzustand übergangen ist; und
Veranlassen der Stimulationserzeugungsschaltlogik (202), die elektrische Stimulation, die den ersten Parametersatz aufweist, abzugeben oder die elektrische Stimulation, die den zweiten Parametersatz aufweist, basierend auf der Bestimmung, ob der Patient in den ersten Patientenzustand übergegangen ist, abzugeben.

5. System nach Anspruch 4,
wobei, vor dem Auswählen des Abfragetherapiemodus mit dem geschlossenen Regelkreis, die Verarbeitungsschaltlogik (210) konfiguriert ist zum:
Verringern einer Intensität der elektrischen Stimulation; und
Bestimmen, dass die elektrische Stimulation, die die verringerte Intensität aufweist, nicht therapeutisch ist, und
wobei, um den Abfragetherapiemodus mit dem geschlossenen Regelkreis auszuwählen, die Verarbeitungsschaltlogik (210) konfiguriert ist, um den Abfragetherapiemodus mit dem geschlossenem Regelkreis, basierend auf der Bestimmung, dass die elektrische Stimulation, die eine verringerte Intensität aufweist, nicht therapeutisch ist, auszuwählen.

6. System nach einem der vorstehenden Ansprüche, wobei die erste Instanz des Biomarker-Signals und die zweite Instanz des Biomarker-Signals eines von einem lokalen Feldpotentialsignal (LFP-Signal), einem evozierten Verbindungsaktionspotentialsignal (ECAP-Signal) und einem Elektromyographiesignal (EMG-Signal) umfassen.

7. System nach einem der vorstehenden Ansprüche, das System ferner umfassend eine implantierbare medizinische Vorrichtung (IMD), wobei die IMD die Stimulationserzeugungsschaltlogik (202), die Erfassungsschaltlogik (204) und die Verarbeitungsschaltlogik (210) einschließt.

8. System nach einem der Ansprüche 1 bis 6, das System ferner umfassend eine implantierbare medizinische Vorrichtung (IMD) und einen externen Programmierer, wobei die IMD die Stimulationserzeugungsschaltlogik (202) und die Erfassungsschaltlogik einschließt und wobei der externe Programmierer oder eine Kombination des externen Programmierers und der IMD die Verarbeitungsschaltlogik (210) einschließen.

9. Computerlesbares Speichermedium, das Anweisungen darauf speichert, die, wenn sie ausgeführt werden, einen oder mehrere Prozessoren veranlassen, ein Verfahren für die Abgabe der elektrischen Stimulation durchzuführen, das Verfahren umfassend:
Veranlassen, mit einer Verarbeitungsschaltlogik, einer Stimulationserzeugungsschaltlogik, die elektrische Stimulation mit einem Patienten in einem ersten Patientenzustand abzugeben;
Empfangen, mit der Verarbeitungsschaltlogik, einer ersten Instanz eines Biomarker-Signals, das durch eine Erfassungsschaltlogik in Anwesenheit der elektrischen Stimulation von der Stimulationserzeugungsschaltlogik, die die elektrische Stimulation mit dem Patienten in dem ersten Patientenzustand abgibt, erfasst wird;
Veranlassen, mit der Verarbeitungsschaltlogik, der Stimulationserzeugungsschaltlogik, die elektrische Stimulation mit dem Patienten in einem zweiten Patientenzustand abzugeben;
Empfangen, mit der Verarbeitungsschaltlogik, einer zweiten Instanz des Biomarker-Signals, das durch die Erfassungsschaltlogik in Anwesenheit der elektrischen Stimulation von der Stimulationserzeugungsschaltlogik, die die elektrische Stimulation mit dem Patienten in dem zweiten Patientenzustand abgibt, erfasst wird;
Bestimmen, mit der Verarbeitungsschaltlogik, ob eine Differenz zwischen der ersten Instanz des Biomarker-Signals und der zweiten Instanz des Biomarker-Signals eine Schwelle erfüllt;
Auswählen, mit der Verarbeitungsschaltlogik, eines Therapiemodus für die Abgabe der elektrischen Stimulation, basierend darauf, ob die Differenz zwischen der ersten Instanz des Biomarker-Signals und der zweiten Instanz des Biomarker-Signals die Schwelle erfüllt; und
Veranlassen, mit der Verarbeitungsschaltlogik, der Stimulationserzeugungsschaltlogik, die elektrische Stimulation gemäß dem ausgewählten Therapiemodus abzugeben.

10. Computerlesbares Speichermedium nach Anspruch 9, wobei mindestens eines von:
(1) der erste Patientenzustand ist, dass der Patient sich nicht bewegt, und der zweite Patientenzustand ist, dass der Patient sich bewegt, und
(2) der erste Patientenzustand ist, dass der Patient ein Arzneimittel eingenommen hat und der zweite Patientenzustand einer des stabilen Zustands des Arzneimittels ist oder wenn das Arzneimittel nicht mehr wirkt.

11. Computerlesbares Speichermedium nach einem der Ansprüche 9 oder 10,
wobei das Bestimmen, ob die Differenz zwischen der ersten Instanz des Biomarker-Signals und der zweiten Instanz des Biomarker-Signals die Schwelle erfüllt, das Bestimmen umfasst, dass die Differenz zwischen der ersten Instanz des Biomarker-Signals und der zweiten Instanz des Biomarker-Signals die Schwelle erfüllt,
wobei das Auswählen des Therapiemodus für die Abgabe der elektrischen Stimulation das Auswählen eines Abfragetherapiemodus mit dem normal geschlossenen Regelkreis umfasst, und
wobei das Veranlassen der Stimulationserzeugungsschaltlogik, die elektrische Stimulation gemäß dem ausgewählten Therapiemodus abzugeben, umfasst:
Veranlassen der Stimulationserzeugungsschaltlogik, zwischen dem Abgeben der elektrischen Stimulation, die einen ersten Parametersatz aufweist, basierend darauf, dass der Patient sich in dem ersten Patientenzustand befindet, und dem Abgeben der elektrischen Stimulation, die einen zweiten Parametersatz aufweist, basierend darauf, dass der Patienten sich in dem zweiten Patientenzustand befindet, überzugehen.

12. Computerlesbares Speichermedium nach einem der Ansprüche 9 oder 10, wobei das Bestimmen, ob die Differenz zwischen der ersten Instanz des Biomarker-Signals und der zweiten Instanz des Biomarker-Signals die Schwelle erfüllt, das Bestimmen umfasst, dass die Differenz zwischen der ersten Instanz des Biomarker-Signals und der zweiten Instanz des Biomarker-Signals die Schwelle nicht erfüllt,
wobei das Auswählen des Therapiemodus für die Abgabe der elektrischen Stimulation das Auswählen eines Abfragetherapiemodus mit dem geschlossenen Regelkreis umfasst, und
wobei das Veranlassen der Stimulationserzeugungsschaltlogik, die elektrische Stimulation gemäß dem ausgewählten Therapiemodus abzugeben, umfasst:
Veranlassen der Stimulationserzeugungsschaltlogik, die elektrische Stimulation, die einen ersten Parametersatz aufweist, basierend darauf, dass der Patient sich in dem ersten Patientenzustand befindet, abzugeben;
Bestimmen, dass der Patient in den zweiten Patientenzustand übergegangen ist;
Veranlassen der Stimulationserzeugungsschaltlogik, die elektrische Stimulation, die einen zweiten Parametersatz aufweist, basierend darauf, dass der Patient sich in dem zweiten Patientenzustand befindet, abzugeben;
vorübergehendes Veranlassen der Stimulationserzeugungsschaltlogik, die Abgabe der elektrischen Stimulation einzustellen oder die elektrische Stimulation, die den ersten Parametersatz aufweist, abzugeben, nachdem die Stimulationserzeugungsschaltlogik die elektrische Stimulation, die den zweiten Parametersatz aufweist, abzugeben;
Bestimmen, während der Einstellung der elektrischen Stimulation oder der Stimulationserzeugungsschaltlogik, die die elektrische Stimulation, die den ersten Parametersatz aufweist, abgibt, ob der Patient in den ersten Patientenzustand übergegangen ist; und
Veranlassen der Stimulationserzeugungsschaltlogik, die elektrische Stimulation, die den ersten Parametersatz aufweist, abzugeben, oder die elektrische Stimulation, die den zweiten Parametersatz aufweist, basierend auf der Bestimmung, ob der Patient in den ersten Patientenzustand übergegangen ist, abzugeben.

13. Computerlesbares Speichermedium nach Anspruch 12, ferner umfassend:
vor dem Auswählen des Abfragetherapiemodus mit dem geschlossenen Regelkreis:
Verringern der Intensität der elektrischen Stimulation; und
Bestimmen, dass die elektrische Stimulation, die die verringerte Intensität aufweist, nicht therapeutisch ist, und
wobei das Auswählen des Abfragetherapiemodus mit dem geschlossenen Regelkreis das Auswählen des Abfragetherapiemodus mit dem geschlossenen Regelkreis, basierend auf der Bestimmung, dass die elektrische Stimulation, die eine verringerte Intensität aufweist, nicht therapeutisch ist, umfasst.

14. Computerlesbares Speichermedium nach einem der Ansprüche 9 bis 13, wobei die erste Instanz des Biomarker-Signals und die zweite Instanz des Biomarker-Signals eines von einem lokalen Feldpotentialsignal, (LFP-Signal), einem evozierten Verbindungsaktionspotentialsignal (ECAP-Signal) und einem Elektromyographiesignal (EMG-Signal) umfassen.

15. Computerlesbares Speichermedium nach einem der Ansprüche 9 bis 14, wobei eine implantierbare medizinische Vorrichtung (IMD) die Stimulationserzeugungsschaltlogik, die Erfassungsschaltlogik und die Verarbeitungsschaltlogik einschließt.

## Revendications

1. Système de distribution de stimulation électrique, le système comprenant :
une circuiterie de génération de stimulation (202) configurée pour administrer une stimulation électrique à un patient ;
une circuiterie de détection (204) configurée pour détecter un ou plusieurs signaux de biomarqueur ; et
une circuiterie de traitement (210) configurée pour :
amener la circuiterie de génération de stimulation (202) à administrer une stimulation électrique avec le patient dans un premier état de patient ;
recevoir une première instance d'un signal de biomarqueur du ou des signaux de biomarqueur détectés en présence de la stimulation électrique provenant de la circuiterie de génération de stimulation (202) administrant la stimulation électrique avec le patient dans le premier état de patient ;
amener la circuiterie de génération de stimulation (202) à administrer une stimulation électrique avec le patient dans un second état de patient ;
recevoir une seconde instance du signal de biomarqueur du ou des signaux de biomarqueur détectés en présence de la stimulation électrique provenant de la circuiterie de génération de stimulation (202) administrant la stimulation électrique avec le patient dans le second état de patient ;
déterminer si une différence entre la première instance du signal de biomarqueur et la seconde instance du signal de biomarqueur satisfait à un seuil ;
sélectionner un mode de thérapie pour l'administration de stimulation électrique sur la base du fait que la différence entre la première instance du signal de biomarqueur et la seconde instance du signal de biomarqueur satisfait au seuil ; et
amener la circuiterie de génération de stimulation (202) à administrer une stimulation électrique conformément au mode de thérapie sélectionné.

2. Procédé selon la revendication 1, dans lequel au moins l'un parmi :
(1) le premier état de patient est le patient ne se déplaçant pas, et le second état de patient est le patient en mouvement, et
(2) le premier état de patient est le patient ayant pris un médicament, et le second état de patient est l'un parmi l'état stable du médicament ou lorsque le médicament n'est plus efficace.

3. Système selon l'une quelconque des revendications 1 à 2,
dans lequel pour déterminer si la différence entre la première instance du signal de biomarqueur et la seconde instance du signal de biomarqueur satisfait au seuil, la circuiterie de traitement (210) est configurée pour déterminer que la différence entre la première instance du signal de biomarqueur et la seconde instance du signal de biomarqueur satisfait au seuil,
dans lequel, pour sélectionner le mode de thérapie pour l'administration de stimulation électrique, la circuiterie de traitement (210) est configurée pour sélectionner un mode de thérapie en boucle fermée normale, et
dans lequel, pour amener la circuiterie de génération de stimulation (202) à délivrer une stimulation électrique conformément au mode de thérapie sélectionné, la circuiterie de traitement (210) est configurée pour :
amener la circuiterie de génération de stimulation (202) à effectuer une transition entre l'administration de stimulation électrique ayant un premier ensemble de paramètres sur la base du fait que le patient est dans le premier état de patient et administrant une stimulation électrique ayant un second ensemble de paramètres sur la base du fait que le patient est dans le second état de patient.

4. Système selon l'une quelconque des revendications 1 ou 2, dans lequel pour déterminer si la différence entre la première instance du signal de biomarqueur et la seconde instance du signal de biomarqueur satisfait au seuil, la circuiterie de traitement (210) est configurée pour déterminer que la différence entre la première instance du signal de biomarqueur et la seconde instance du signal de biomarqueur ne satisfait pas au seuil,
dans lequel, pour sélectionner le mode de thérapie pour l'administration de stimulation électrique, la circuiterie de traitement (210) est configurée pour sélectionner un mode de thérapie en boucle fermée de scrutation et
dans lequel, pour amener la circuiterie de génération de stimulation (202) à délivrer une stimulation électrique conformément au mode de thérapie sélectionné, la circuiterie de traitement (210) est configurée pour :
amener la circuiterie de génération de stimulation (202) à administrer une stimulation électrique ayant un premier ensemble de paramètres sur la base du fait que le patient est dans le premier état de patient ;
déterminer que le patient passe au second état de patient ;
amener la circuiterie de génération de stimulation (202) à administrer une stimulation électrique ayant un second ensemble de paramètres sur la base du fait que le patient est dans le second état de patient ;
après que la circuiterie de génération de stimulation (202) administre une stimulation électrique ayant le second ensemble de paramètres, amener temporellement la circuiterie de génération de stimulation (202) à cesser l'administration de stimulation électrique ou à administrer une stimulation électrique ayant le premier ensemble de paramètres ;
lors de la cessation de la stimulation électrique ou de la circuiterie de génération de stimulation (202) administrant une stimulation électrique présentant le premier ensemble de paramètres, déterminer si le patient passe au premier état de patient ; et
amener la circuiterie de génération de stimulation (202) à administrer une stimulation électrique présentant le premier ensemble de paramètres ou à délivrer une stimulation électrique présentant le second ensemble de paramètres sur la base de la détermination si le patient passe au premier état de patient.

5. Système selon la revendication 4,
dans lequel avant de sélectionner le mode de thérapie en boucle fermée de scrutation, la circuiterie de traitement (210) est configurée pour :
réduire l'intensité de la stimulation électrique ; et
déterminer qu'une stimulation électrique ayant l'intensité réduite n'est pas thérapeutique, et
dans lequel pour sélectionner le mode de thérapie en boucle fermée de scrutation, la circuiterie de traitement (210) est configurée pour sélectionner le mode de thérapie en boucle fermée de scrutation sur la base de la détermination que la stimulation électrique ayant une intensité réduite n'est pas thérapeutique.

6. Système selon l'une quelconque des revendications précédentes, dans lequel la première instance du signal de biomarqueur et la seconde instance du signal de biomarqueur comprennent l'un parmi un signal de potentiel de champs locaux (LFP), un signal de potentiel d'action composé évoqué (ECAP) et un signal d'électromyographie (EMG).

7. Système selon l'une quelconque des revendications précédentes, le système comprenant en outre un dispositif médical implantable (IMD), dans lequel l'IMD comporte la circuiterie de génération de stimulation (202), la circuiterie de détection (204) et la circuiterie de traitement (210).

8. Système selon l'une quelconque des revendications 1 à 6, le système comprenant en outre un dispositif médical implantable (IMD) et un programmateur externe, dans lequel l'IMD comporte la circuiterie de génération de stimulation (202) et la circuiterie de détection, et dans lequel le programmateur externe ou une combinaison du programmateur externe et de l'IMD comporte la circuiterie de traitement (210).

9. Support de stockage lisible par ordinateur stockant des instructions sur celui-ci, qui lorsqu'elles sont exécutées amènent un ou plusieurs processeurs à réaliser un procédé d'administration de stimulation électrique, le procédé comprenant :
le fait d'amener, avec une circuiterie de traitement, une circuiterie de génération de stimulation à administrer une stimulation électrique avec un patient dans un premier état de patient ;
la réception, avec la circuiterie de traitement, d'une première instance d'un signal de biomarqueur détecté par une circuiterie de détection en présence de la stimulation électrique provenant de la circuiterie de génération de stimulation administrant la stimulation électrique avec le patient dans le premier état de patient ;
le fait d'amener, avec la circuiterie de traitement, la circuiterie de génération de stimulation à administrer une stimulation électrique avec le patient dans un second état de patient ;
la réception, avec la circuiterie de traitement, d'une seconde instance du signal de biomarqueur détecté par la circuiterie de détection en présence de la stimulation électrique provenant de la circuiterie de génération de stimulation administrant la stimulation électrique avec le patient dans le second état de patient ;
la détermination, avec la circuiterie de traitement, si une différence entre la première instance du signal de biomarqueur et la seconde instance du signal de biomarqueur satisfait à un seuil ;
la sélection, avec la circuiterie de traitement, d'un mode de thérapie pour l'administration de stimulation électrique sur la base du fait que la différence entre la première instance du signal de biomarqueur et la seconde instance du signal de biomarqueur satisfait au seuil ; et
le fait d'amener, avec la circuiterie de traitement, la circuiterie de génération de stimulation à administrer une stimulation électrique conformément au mode de thérapie sélectionné.

10. Support de stockage lisible par ordinateur selon la revendication 9, dans lequel au moins l'un parmi :
(1) le premier état de patient est le patient ne se déplaçant pas, et le second état de patient est le patient en mouvement, et
(2) le premier état de patient est le patient ayant pris un médicament, et le second état de patient est l'un parmi l'état stable du médicament ou lorsque le médicament n'est plus efficace.

11. Support de stockage lisible par ordinateur selon l'une quelconque des revendications 9 ou 10,
dans lequel la détermination si la différence entre la première instance du signal de biomarqueur et la seconde instance du signal de biomarqueur satisfait au seuil comprend la détermination que la différence entre la première instance du signal de biomarqueur et la seconde instance du signal de biomarqueur satisfait au seuil,
dans lequel la sélection du mode de thérapie pour l'administration de stimulation électrique comprend la sélection d'un mode de thérapie en boucle fermée normale, et
dans lequel le fait d'amener la circuiterie de génération de stimulation à délivrer une stimulation électrique conformément au mode de thérapie sélectionné comprend :
le fait d'amener la circuiterie de génération de stimulation à effectuer une transition entre la délivrance d'une stimulation électrique ayant un premier ensemble de paramètres sur la base du fait que le patient est dans le premier état de patient et à administrer une stimulation électrique ayant un second ensemble de paramètres sur la base du fait que le patient est dans le second état de patient.

12. Support de stockage lisible par ordinateur selon l'une quelconque des revendications 9 ou 10, dans lequel la détermination si la différence entre la première instance du signal de biomarqueur et la seconde instance du signal de biomarqueur satisfait au seuil comprend la détermination que la différence entre la première instance du signal de biomarqueur et la seconde instance du signal de biomarqueur ne satisfait pas au seuil,
dans lequel la sélection du mode de thérapie pour l'administration de stimulation électrique comprend la sélection d'un mode de thérapie en boucle fermée de scrutation, et
dans lequel le fait d'amener la circuiterie de génération de stimulation à délivrer une stimulation électrique conformément au mode de thérapie sélectionné comprend :
le fait d'amener la circuiterie de génération de stimulation à administrer une stimulation électrique ayant un premier ensemble de paramètres sur la base du fait que le patient est dans le premier état de patient ;
la détermination que le patient est passé au second état de patient ;
le fait d'amener la circuiterie de génération de stimulation à administrer une stimulation électrique ayant un second ensemble de paramètres sur la base du fait que le patient est dans le second état de patient ;
après que la circuiterie de génération de stimulation administre une stimulation électrique ayant le second ensemble de paramètres, le fait d'amener temporellement la circuiterie de génération de stimulation à cesser l'administration de stimulation électrique ou à administrer une stimulation électrique ayant le premier ensemble de paramètres ;
lors de la cessation de la stimulation électrique ou de la circuiterie de génération de stimulation administrant une stimulation électrique ayant le premier ensemble de paramètres, la détermination de savoir si le patient est passé au premier état de patient ; et
le fait d'amener la circuiterie de génération de stimulation à administrer une stimulation électrique ayant le premier ensemble de paramètres ou administrer une stimulation électrique ayant le second ensemble de paramètres sur la base de la détermination si le patient est passé au premier état de patient.

13. Support de stockage lisible par ordinateur selon la revendication 12, comprenant en outre :
avant de sélectionner le mode de thérapie en boucle fermée de scrutation :
réduire l'intensité de la stimulation électrique ; et
la détermination qu'une stimulation électrique ayant une intensité réduite n'est pas thérapeutique, et
dans lequel la sélection du mode de thérapie en boucle fermée de scrutation comprend la sélection du mode de thérapie en boucle fermée de scrutation sur la base de la détermination que la stimulation électrique ayant une intensité réduite n'est pas thérapeutique.

14. Support de stockage lisible par ordinateur selon l'une quelconque des revendications 9 à 13, dans lequel la première instance du signal de biomarqueur et la seconde instance du signal de biomarqueur comprennent l'un des éléments suivants : un signal de potentiel de champs locaux (LFP), un signal de potentiel d'action composé évoqué (ECAP) et un signal d'électromyographie (EMG).

15. Support de stockage lisible par ordinateur selon l'une quelconque des revendications 9 à 14, dans lequel un dispositif médical implantable (IMD) comporte la circuiterie de génération de stimulation, la circuiterie de détection et la circuiterie de traitement.
